(19) Europäisches Patentamt
European Patent Office
Office européen des brevets

(11) EP 4 321 525 A1

(12) EUROPEAN PATENT APPLICATION
published in accordance with Art. 153(4) EPC

(43) Date of publication:
**14.02.2024 Bulletin 2024/07**

(21) Application number: **22778770.2**

(22) Date of filing: **25.03.2022**

(51) International Patent Classification (IPC):
***C07K 7/06*** *(2006.01)* ***C07K 5/117*** *(2006.01)*
***A61K 38/08*** *(2019.01)* ***A61K 38/07*** *(2006.01)*
***A61P 5/06*** *(2006.01)*

(52) Cooperative Patent Classification (CPC):
Y02P 20/55

(86) International application number:
**PCT/CN2022/082973**

(87) International publication number:
**WO 2022/206587 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2021 CN 202110343062**

(71) Applicant: **Chengdu Sintanovo Biotechnology Co., Ltd**
**Chengdu Sichuan 610000 (CN)**

(72) Inventors:
- **MENG, Guangpeng**
  **Chengdu, Sichuan 610000 (CN)**
- **FU, Xiaoping**
  **Chengdu, Sichuan 610000 (CN)**
- **LI, Sijun**
  **Chengdu, Sichuan 610000 (CN)**
- **MA, Haoyu**
  **Chengdu, Sichuan 610000 (CN)**
- **ZHANG, Yin**
  **Chengdu, Sichuan 610000 (CN)**
- **LIU, Shuang**
  **Chengdu, Sichuan 610000 (CN)**
- **XU, Miao**
  **Chengdu, Sichuan 610000 (CN)**
- **GAO, Jian**
  **Chengdu, Sichuan 610000 (CN)**
- **LI, Yuanbo**
  **Chengdu, Sichuan 610000 (CN)**

(74) Representative: **Studio Torta S.p.A.**
**Via Viotti, 9**
**10121 Torino (IT)**

# (54) POLYPEPTIDE COMPOUND AND APPLICATION THEREOF

(57) A polypeptide compound having a structure shown in formula I, or a stereoisomer, mixture or pharmaceutically acceptable salt thereof. Experimental results show that the polypeptide compound can effectively exhibit high agonistic activity to GHSR-1a.

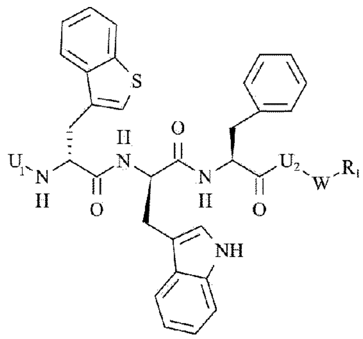

formula I.

EP 4 321 525 A1

## Description

The present application claims priority to Chinese Patent Application No. 202110343062.2 filed with China National Intellectual Property Administration on Mar. 30, 2021 and titled "POLYPEPTIDE COMPOUND AND APPLICATION THEREOF", which is incorporated herein by reference in its entirety.

## TECHNICAL FIELD

The present invention relates to the technical field of biomedicine and particularly to a polypeptide compound and use thereof.

## BACKGROUND

Ghrelin is an endogenous ligand for growth hormone secretagogue receptor (GHSR). Ghrelin was found by Kojima et al. in mouse and human gastric endocrine cells and the arcuate nucleus of the hypothalamus. It is the only natural ligand that has been found as yet for GHSR. It contains 28 amino acid residues and has a molecular weight of 3.3 kDa. The human and rat ghrelin precursor proteins consist of 117 amino acids, and the first N-terminal tricosapeptide has the characteristics of a secretory signal peptide; the fragment of the first 4 N-terminal amino acids of ghrelin is its smallest active center, and the C-terminal P-R structure (proline-arginine) is its recognition site. Human ghrelin and rat ghrelin differ in only 2 amino acid residues, and the coding gene sequences have 82.9% homology. Ghrelin is secreted *in vivo* in two forms: a form in which the serine at position 3 of the N-terminus of ghrelin is octanoylated, and a form in which the serine is not octanoylated. The serine at position 3 of the N-terminus of ghrelin is the essential part that fulfills its biological function. In early studies, des-acyl ghrelin was believed to not have biological activity. However, recent studies show that both des-acyl ghrelin and acyl ghrelin can promote the proliferation of the notochordal neuroepithelium; des-acyl ghrelin has an endocrine function, can promote cell proliferation and plays an anti-apoptotic role.

GHSR is an orphan nuclear G protein-coupled receptor that mainly exists in the pituitary and stomach of rodents and humans. It is also widely distributed in peripheral tissues, the brain, the intestines, the kidneys, the pancreas, the heart, adipose tissues, and the like. The wide distribution of GHSR plays an important role in multiple biological functions of ghrelin and its receptors. The structural coding genome of GHSR is highly conserved in different species, and its amino acid sequence has 52% homology with the G protein-coupled protein receptor of the motilin gene-related peptide. GHSR is divided by exon codes into type la and type lb. GHSR-la is a functional receptor of ghrelin, which, upon binding to ghrelin, activates phospholipase C (PLC), inositol trisphosphate (IP3), protein kinase C (PKC), and the like to produce biological effects. The non-functional receptor GHSR-lb has no biological activity.

The pulsatile release of growth hormone (GH) in the pituitary is regulated by three major factors: growth hormone releasing hormone (GHRH), somatostatin (SS) and ghrelin in the hypothalamus. On GH secretion, GHRH has a promoting effect, SS has an inhibitory effect, and ghrelin and GHRH can synergistically have a promoting effect. The three of them form a local neuroendocrine regulation feedback loop in the hypothalamus. In a system that regulates GH secretion, GHRH binds to its receptor, increasing the level of intracellular cyclic adenosine monophosphate; ghrelin binds to its receptor, leading to $K^+$ channel depolarization and inhibition, causing an increase in the concentration of intracellular IP3 and an increase in the concentration of intracellular $Ca^{2+}$, and finally stimulating GH secretion. The release of GH from somatotropic cells in the pituitary can also be controlled by growth hormone-releasing peptides (GHRPs). It has been found that there is a hexapeptide, H-His-D-Trp-Ala-Trp-D-Phe-Lys-$CONH_2$ (GHRP-6), that regulates growth hormone release in somatotropic cells in a dose-dependent manner in several species including humans (Bowers et. al., Endocrinology 1984,114,1537-1545). By analyzing the structure of GHRP-6, researchers also discovered some GHRP analogs.

Such polypeptides and compounds such as peptoids can bind to GHSR-la, producing agonistic activity, causing signal transduction and thereby regulating GH secretion. However, all these compounds have certain limitations in clinical development. Therefore, the research on GHSR-la receptor agonists aims to develop structures with high activity, for use in low doses and with low toxic and side effects.

It is one of the effective drug development strategies to improve the stability of a polypeptide while keeping or improving the physiological activity of the polypeptide.

## SUMMARY

In view of this, the technical problem to be addressed by the present invention is to provide a polypeptide compound and use thereof, wherein the prepared polypeptide compound has high activity as a GHSR-la receptor agonist.

To achieve the aim described above, the present invention provides a polypeptide compound having a structure represented by formula I, or a stereoisomer, mixture or pharmaceutically acceptable salt thereof:

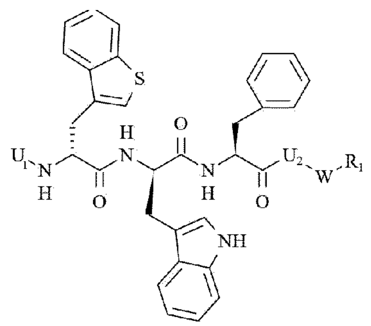

formula I;

wherein,

$R_1$ is selected from $-NR_2R_3$, $-OR_2$ and $-SR_2$;

and, $R_1$ is not a D- or L-amino acid;

$R_2$ and $R_3$ are independently selected from hydrogen, deuterium, a polymer derived from polyethylene glycol, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl.

W is selected from a single bond, a D-amino acid and an L-amino acid;

$U_1$ is selected from any one of the following structures:

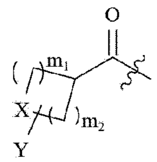 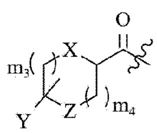 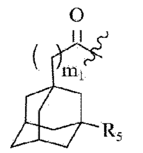 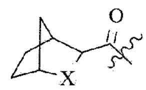 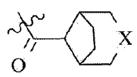 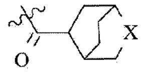

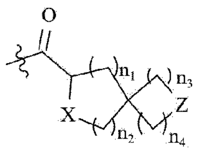 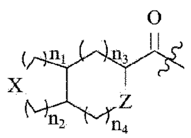 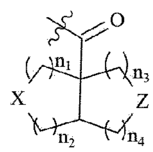 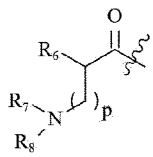 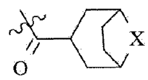 ;

wherein X and Z are independently selected from $CH-R_4$, $N-R_4$, O, S, Se, S=O and O=S=O;

$R_4$, $R_7$ and $R_8$ are independently selected from hydrogen, deuterium, amino, a protective group, a polymer derived from polyethylene glycol, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, substituted or unsubstituted aralkyl, and $R_9CO-$;

Y is selected from halogen, amino, nitro, hydroxyl and cyano;

$R_5$ is selected from $-NR_2R_3$, $-OR_2$ and $-SR_2$;

$R_6$ is selected from hydrogen, deuterium, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl;

$m_1$ and $m_2$ are independently selected from 0, 1, 2 and 3; particularly, when X is N, $m_1$ is 2, and $m_2$ is then independently selected from 0, 1 and 3; $m_2$ is 2, and $m_1$ is then independently selected from 0, 1 and 3;

$m_3$ and $m_4$ are independently selected from 0, 1, 2 and 3;

$n_1$, $n_2$, $n_3$ and $n_4$ are independently selected from 0, 1, 2 and 3;

p is 0, 1, 2, 3, 4 or 5;

$U_2$ is a single bond, or is selected from any one of the following structures, and the carbonyl end of $U_2$ is linked to W:

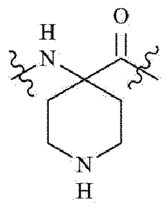 (Apc), 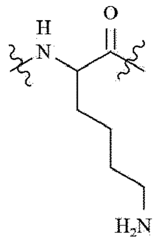 (Lys), 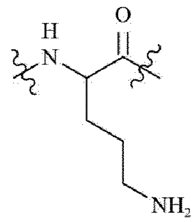 (Orn) and 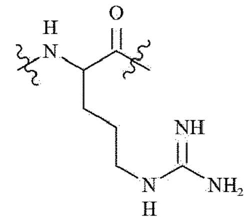

(Arg);

$R_9$ is selected from hydrogen, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl.

In the present invention, the $R_1$ is selected from $-NR_2R_3$, $-OR_2$ and $SR_2$,
wherein $R_2$ and $R_3$ are independently selected from hydrogen, deuterium, a polymer derived from polyethylene glycol, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl; more preferably hydrogen, deuterium, a polymer derived from polyethylene glycol, an acyclic substituted or unsubstituted $C_{1-10}$ aliphatic group, a substituted or unsubstituted $C_{3-10}$ alicyclic group, substituted or unsubstituted $C_{2-10}$ heterocyclyl, substituted or unsubstituted $C_{2-20}$ heteroarylalkyl, substituted or unsubstituted $C_{6-12}$ aryl, and substituted or unsubstituted $C_{6-12}$ aralkyl.

In the present invention, the $R_1$ is preferably selected from $-NR_2R_3$ and $-OR_2$, wherein $R_2$ and $R_3$ are independently selected from hydrogen, methyl, ethyl, hexyl, dodecyl and hexadecyl.

In the present invention, the $R_1$ is not a D- or L-amino acid.

In the present invention, the amino acid described above refers to an amino acid residue, specifically a residue after a polypeptide is formed by amino acids reacting via amino or carboxyl.

In the present invention, the W is independently selected from a single bond, a D-amino acid and an L-amino acid. More preferably, the W is selected from one or more of a single bond and alanine, arginine, asparagine, cysteine, glutamine, aspartic acid, glutamic acid, glycine, histidine, isoleucine, leucine, lysine, methionine, phenylalanine, proline, serine, threonine, tryptophan, tyrosine and valine residues.

In the present invention, when the W is selected from a single bond, $U_2$ and $R_1$ are directly linked.

When the W is selected from a D-amino acid and an L-amino acid, the amino acid loses one molecule of water and forms an amido bond with an adjacent group.

The residue refers to a case where W forms an amido bond with an adjacent group by losing one molecule of water and a polypeptide compound is thus formed.

In the present invention, the $U_1$ is selected from any one of the following structures:

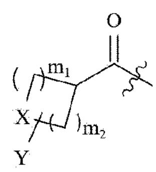 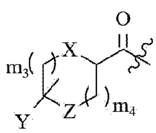 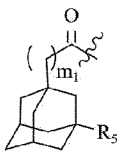 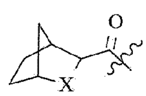 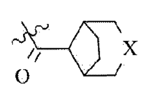 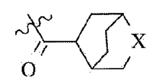

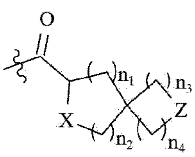 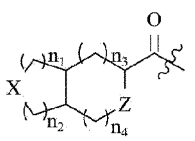 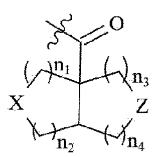 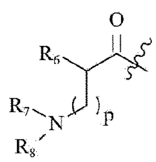 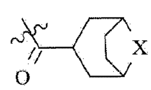

wherein X and Z are independently selected from $CH-R_4$, $N-R_4$, O, S, Se, S=O and O=S=O; more preferably $N-R_4$ and O.

$R_4$, $R_7$ and $R_8$ are independently selected from hydrogen, deuterium, amino, a protective group, a polymer derived from polyethylene glycol, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, substituted or unsubstituted aralkyl, and $R_9CO-$; more preferably hydrogen, deuterium, amino, a

polymer derived from polyethylene glycol, an acyclic substituted or unsubstituted $C_{1-10}$ aliphatic group, a substituted or unsubstituted $C_{3-10}$ alicyclic group, substituted or unsubstituted $C_{2-10}$ heterocyclyl, substituted or unsubstituted $C_{2-20}$ heteroarylalkyl, substituted or unsubstituted $C_{6-12}$ aryl, substituted or unsubstituted $C_{6-12}$ aralkyl, and $R_9CO$-; further preferably hydrogen, amino, $C_{1-6}$ alkyl, $C_{6-14}$ aryl, $C_{3-8}$ cycloalkyl and $C_{2-10}$ acyl.

The $R_9$ is preferably hydrogen, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, or substituted or unsubstituted aralkyl; more preferably hydrogen, an acyclic substituted or unsubstituted $C_{1-10}$ aliphatic group, a substituted or unsubstituted $C_{3-10}$ alicyclic group, substituted or unsubstituted $C_{2-10}$ heterocyclyl, substituted or unsubstituted $C_{2-20}$ heteroarylalkyl, substituted or unsubstituted $C_{6-12}$ aryl, or substituted or unsubstituted $C_{6-12}$ aralkyl; further preferably hydrogen and $C_{1-6}$ alkyl; in some specific examples of the present invention, the $R_9$ is specifically methyl, ethyl, propyl, isopropyl or butyl.

In the present invention, the Y is selected from halogen, amino, nitro, hydroxyl and cyano; more preferably F, Cl, Br and amino.

In the present invention, the $R_5$ is independently selected from $-NR_2R_3$, $-OR_2$ and $-SR_2$; more preferably $-NR_2R_3$.

The ranges for the $R_2$ and $R_3$ described above are the same as described above and are not described herein again.

Further preferably, $R_2$ and $R_3$ are independently selected from hydrogen, methyl, ethyl and hexyl.

In the present invention, the $R_6$ is independently selected from hydrogen, deuterium, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl; more preferably hydrogen, deuterium, an acyclic substituted or unsubstituted $C_{1-10}$ aliphatic group, a substituted or unsubstituted $C_{3-10}$ alicyclic group, substituted or unsubstituted $C_{2-10}$ heterocyclyl, substituted or unsubstituted $C_{2-20}$ heteroarylalkyl, substituted or unsubstituted $C_{6-20}$ aryl, and substituted or unsubstituted $C_{6-12}$ aralkyl; further preferably hydrogen, $C_{1-6}$ alkyl, $C_{6-14}$ aryl and $C_{3-8}$ cycloalkyl.

In the present invention, the $U_1$ is preferably selected from the following structures:

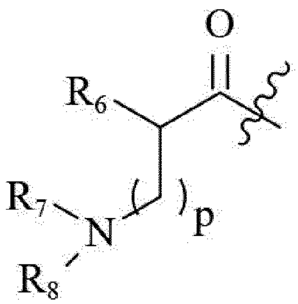
;

wherein $R_6$ is selected from hydrogen, substituted or unsubstituted $C_{1-6}$ alkyl, substituted or unsubstituted $C_{6-14}$ aryl, and substituted or unsubstituted $C_{3-8}$ cycloalkyl; the substituted group is preferably halogen, amino, nitro, hydroxyl, acyl-substituted amino, ureido or guanidino.

Further preferably, the $R_6$ is selected from hydrogen and substituted or unsubstituted methyl, ethyl, propyl, isopropyl, butyl, isobutyl or tert-butyl.

The substituted group is selected from halogen, amino, nitro, hydroxyl, formamido, acetamido, propionamido, butyramido, ureido and guanidino.

$R_7$ and $R_8$ are independently and preferably hydrogen, $C_{1-6}$ alkyl, $C_{6-14}$ aryl, $C_{3-8}$ cycloalkyl or $C_{2-10}$ acyl; more preferably hydrogen, methyl, ethyl, propyl, isopropyl, butyl, isobutyl, tert-butyl, formyl, acetyl, propionyl or butyryl.

In the present invention, the $U_1$ is preferably selected from any one of the following structures:

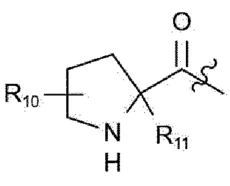 , 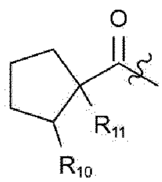 , 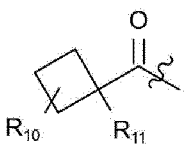 , 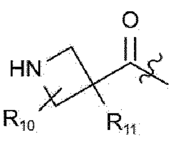 , 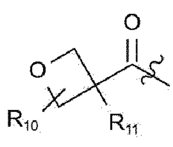 ,

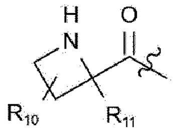, 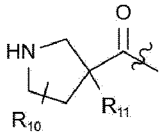, 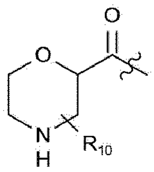, 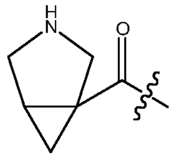, 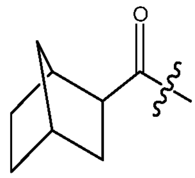,

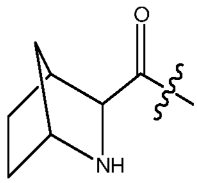 and 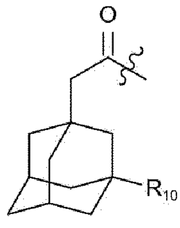;

wherein $R_{10}$ and $R_{11}$ are independently and preferably hydrogen, amino, nitro, hydroxyl, halogen, cyano, aminomethyl, aminoethyl, aminopropyl or aminobutyl.

In the present invention, the $U_2$ is preferably a single bond, or is selected from any one of the following structures (i.e., 4-amino-4-piperidinecarboxylic acid (Apc) or lysine (Lys), ornithine (Orn) and arginine (Arg) residues), and the carbonyl end of $U_2$ is linked to W:

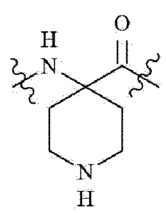 (Apc), 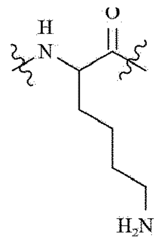 (Lys), 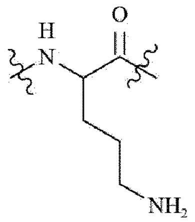 (Orn) and 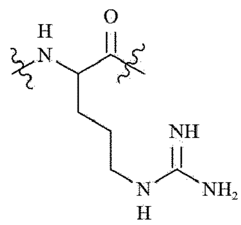

(Arg).

In the present invention, when $U_2$ is a single bond, W is directly linked to the carbonyl group of the parent core.

In the present invention, the $m_1$ and $m_2$ are independently selected from 0, 1, 2 and 3; particularly, when X is N, $m_1$ is 2, and $m_2$ is then independently selected from 0, 1 and 3; $m_2$ is 2, $m_1$ is then independently selected from 0, 1 and 3; that is, the structure comprising $m_1$ and $m_2$ is not selected from pyridinyl groups.

In the present invention, $m_3$ and $m_4$ are independently selected from 0, 1, 2 and 3. In the present invention, $n_1$, $n_2$, $n_3$ and $n_4$ are independently selected from 0, 1, 2 and 3.

In the present invention, p is 0, 1, 2, 3, 4 or 5.

In the present invention, when p is 0, the N atom is directly linked to a C atom.

In the present invention, the polypeptide compound preferably has any one of the following structures, or a stereoisomer, mixture or pharmaceutically acceptable salt thereof:

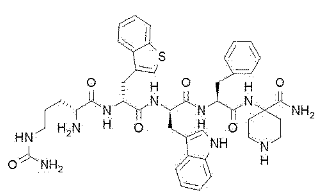, 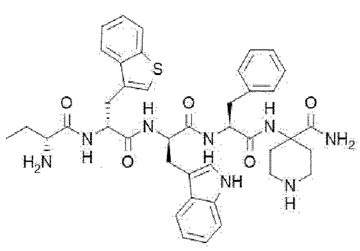, 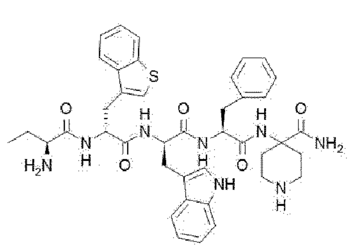,

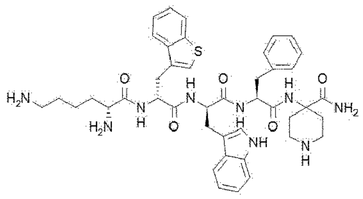,
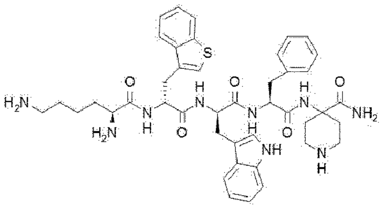,
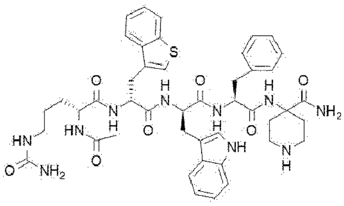,

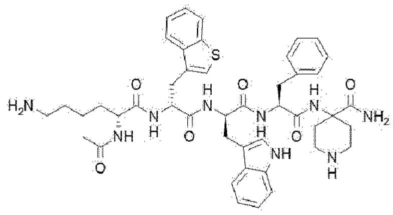,
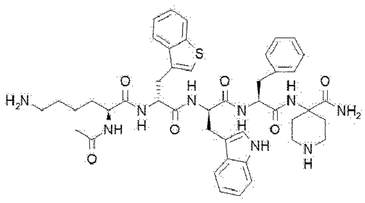,
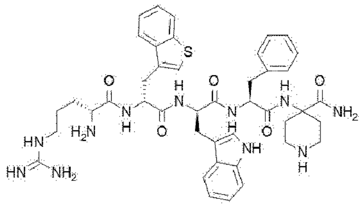,

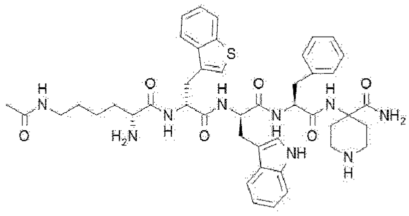,
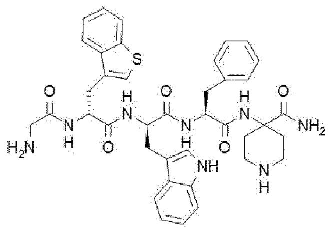,
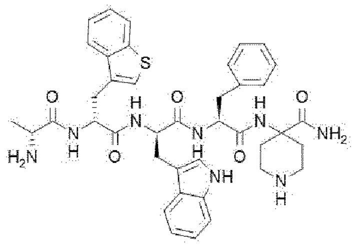,

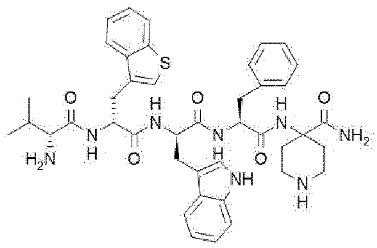,
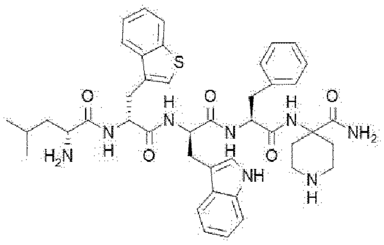,
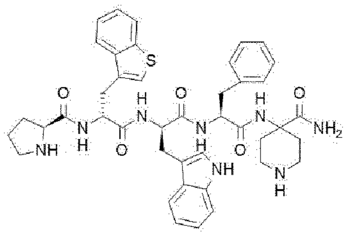,

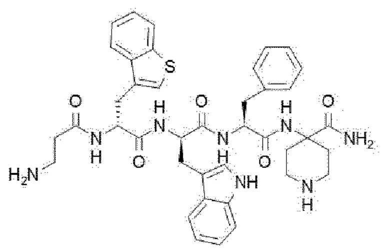,
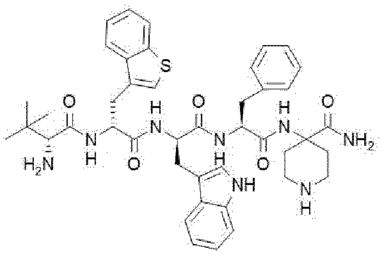,
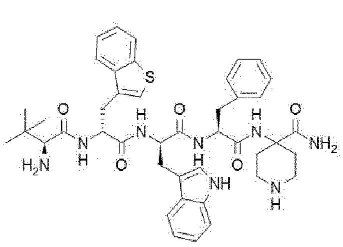,

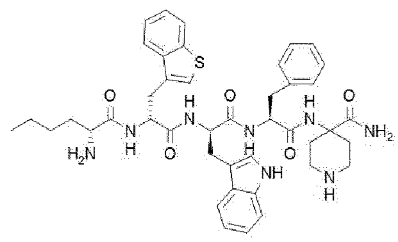,
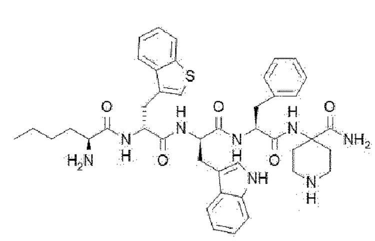,
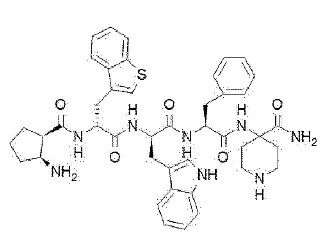,

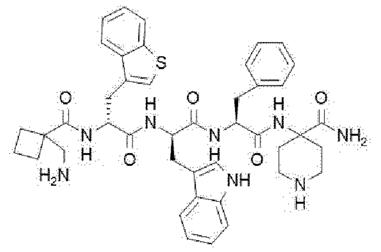,
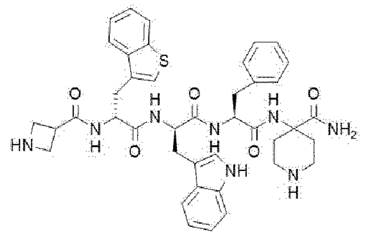,
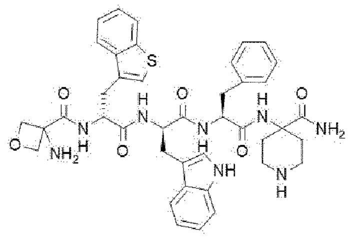,

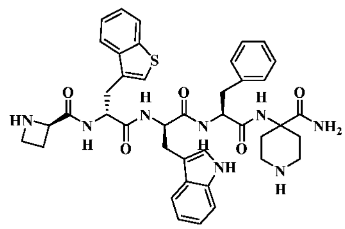,
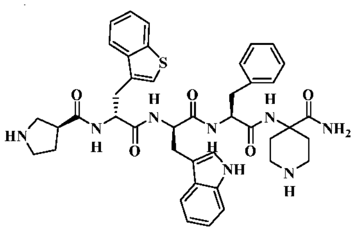,
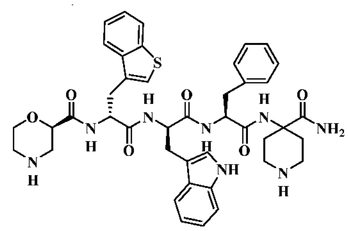,

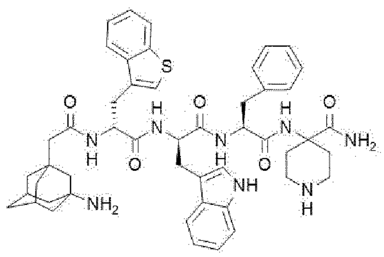,
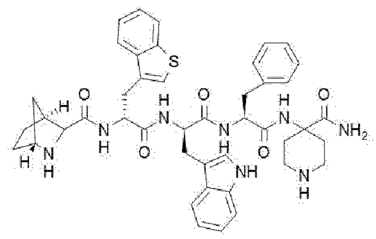,
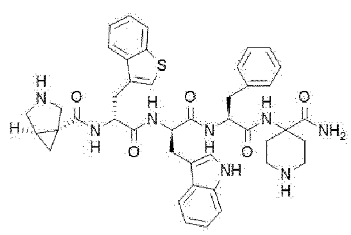,

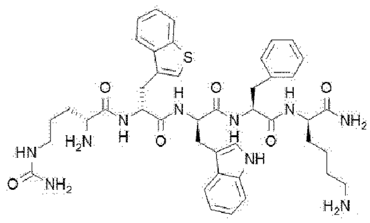,
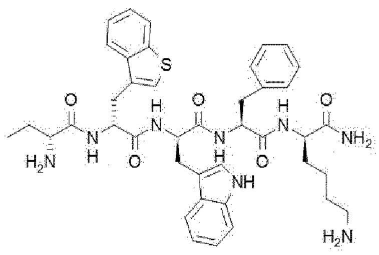,
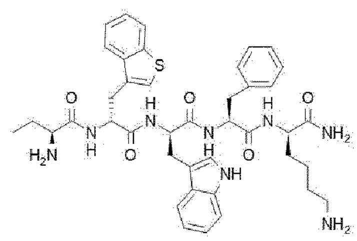,

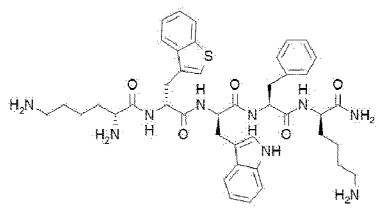,
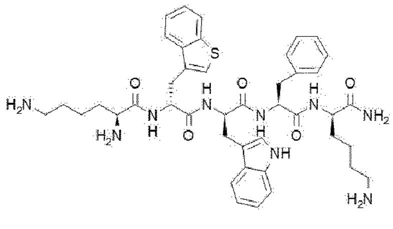,
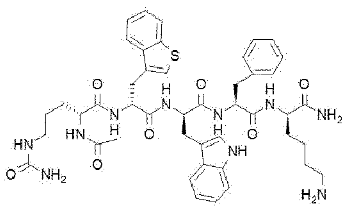,

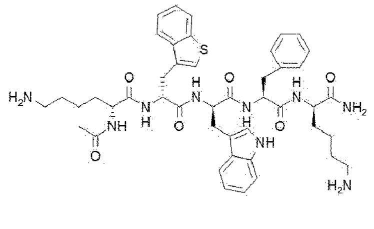,
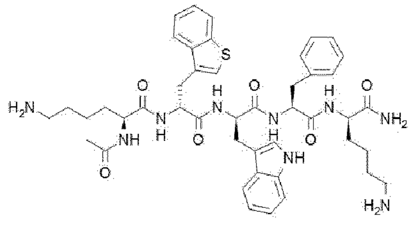,
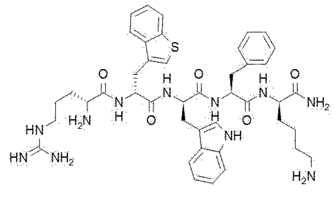,

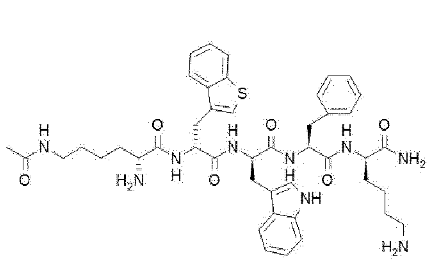,

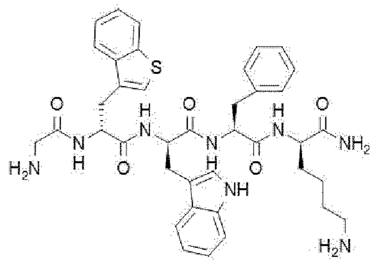,

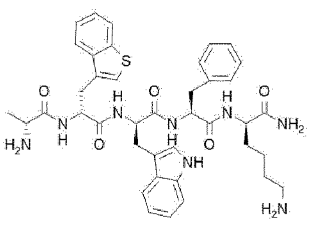,

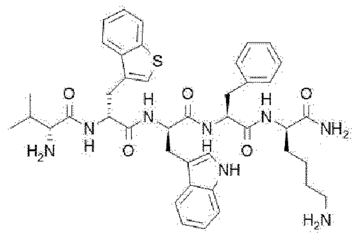,

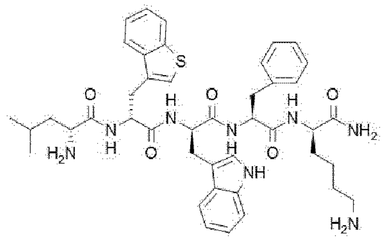,

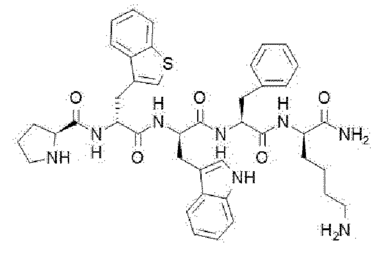,

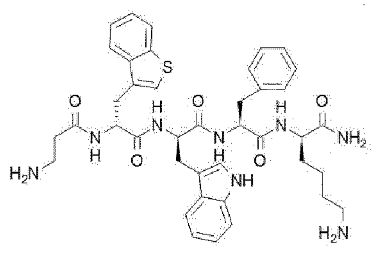,

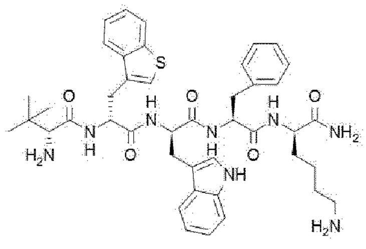,

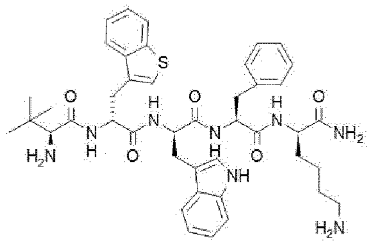,

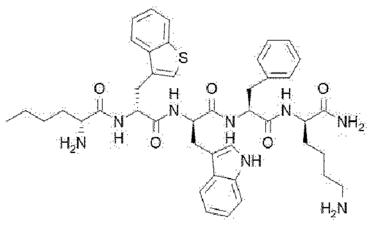,

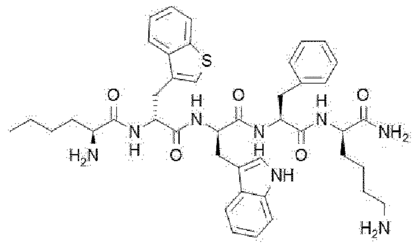,

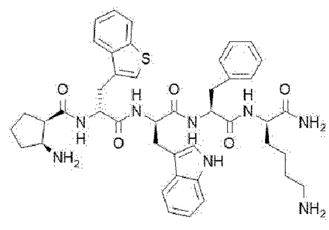,

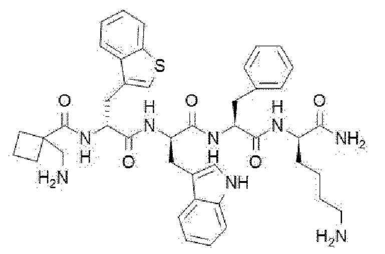,

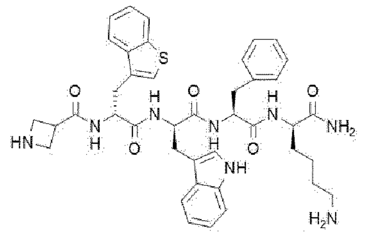,

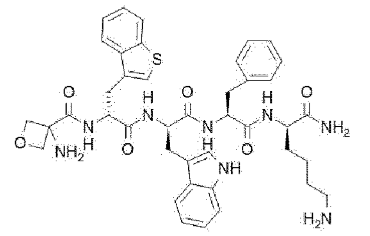,

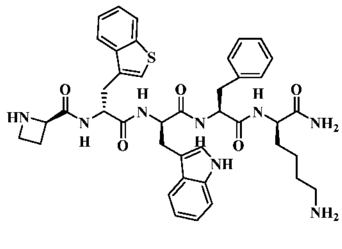,

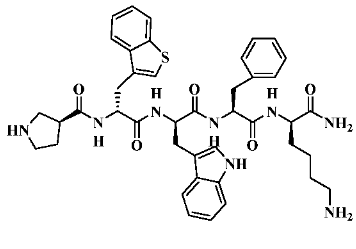,

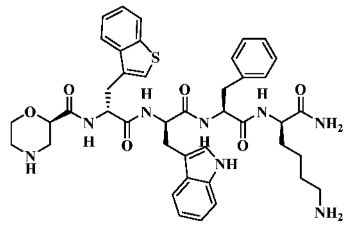,

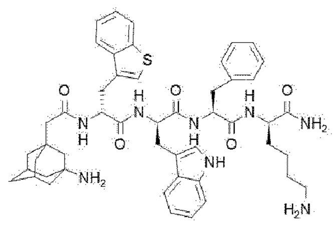,
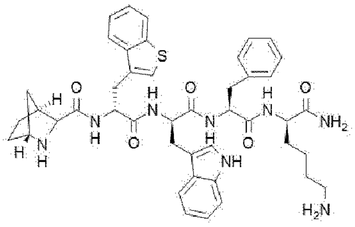,
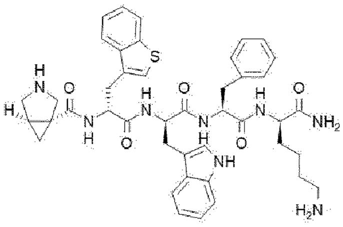,

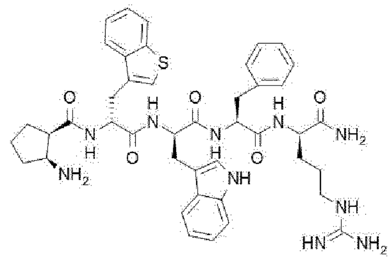,
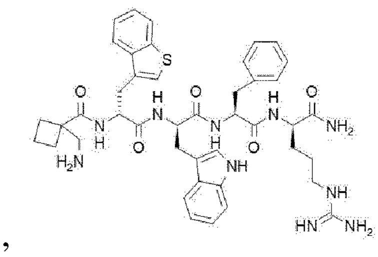,
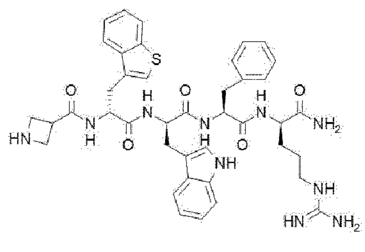,

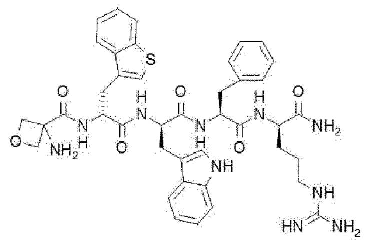,
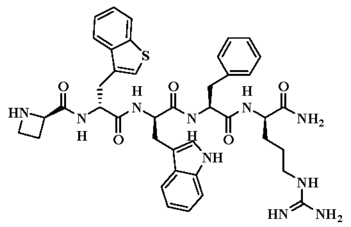,
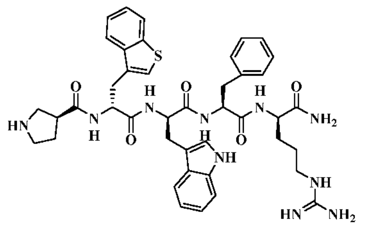,

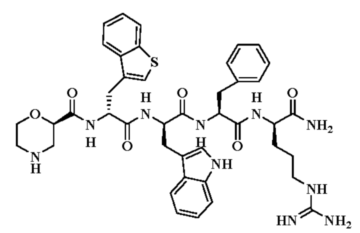,
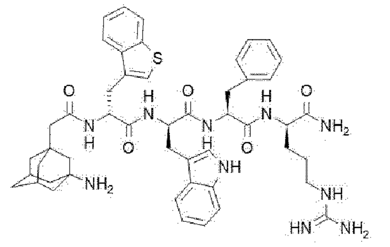,
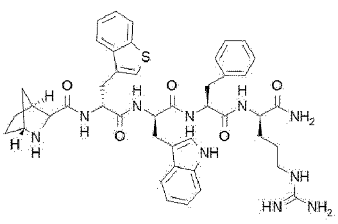,

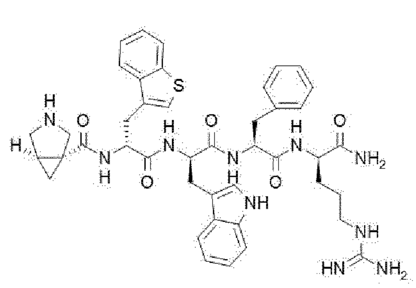,
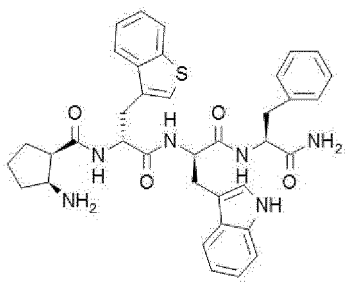,
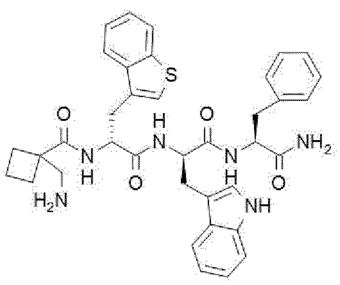,

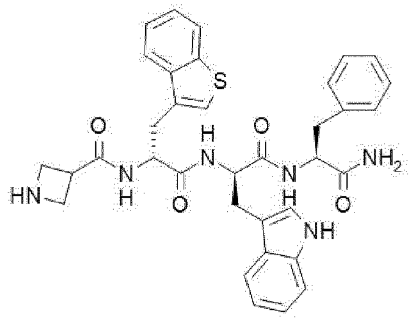,
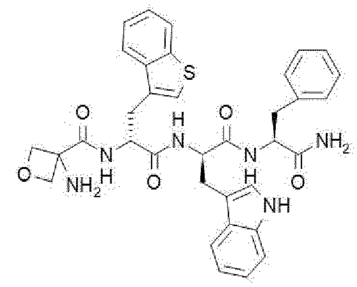,
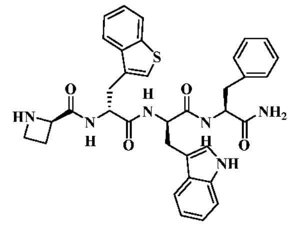,

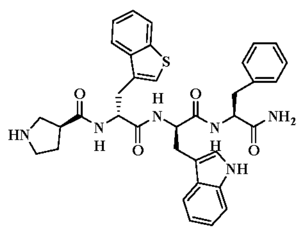,
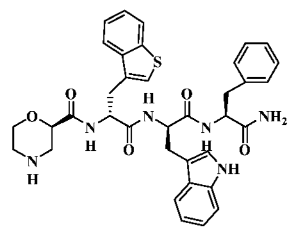,
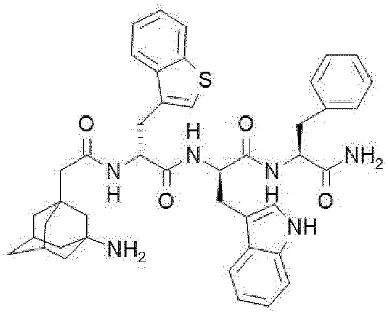,

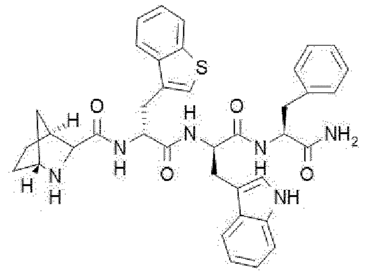,
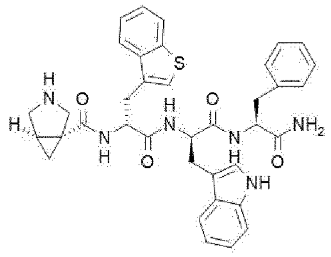,
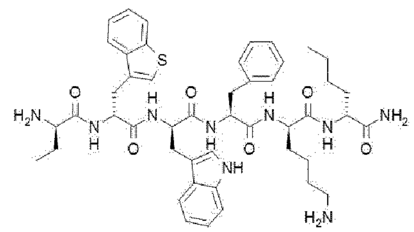,

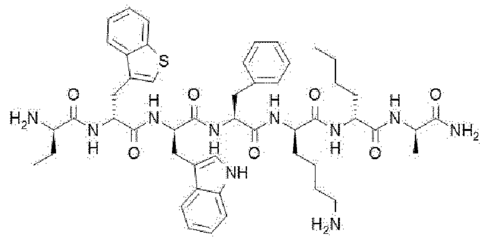
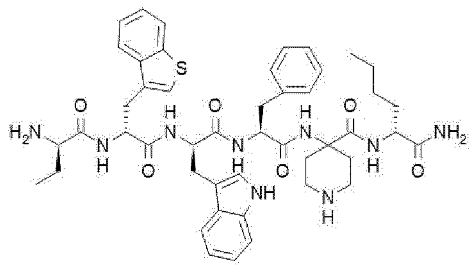,

and

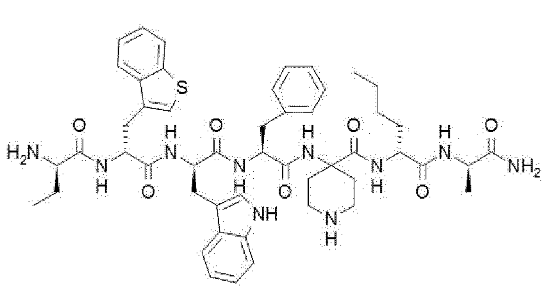.

In an embodiment of the present invention, the synthesis of the polypeptide compounds described herein, their

stereoisomers, mixtures thereof and their pharmaceutically acceptable salts can be performed according to any conventional method known in the prior art, such as using solid phase peptide synthesis methods [Stewart J. M. y Young J. D., "Solid Phase Peptide Synthesis, 2nd edition", (1984), Pierce Chemical Company, Rockford, Illinois; Bodanzsky M. y Bodanzsky A., "The practice of Peptide Synthesis", (1994), Springer Verlag, Berlin; Lloyd Williams P. et al., "Chemical Approaches to the Synthesis of Peptides and Proteins", (1997), CRC, Boca Raton, FL, USA], synthesis in solution, enzymic synthesis [Kullmann W. "Proteases as catalysts for enzymic syntheses of opioid peptides", (1980), J. Biol. Chem., 255(17), 8234-8238] or any combination thereof. Compounds can also be obtained by fermentation of a strain of bacteria modified or unmodified by genetic engineering with the objective of producing the desired sequences, or by controlled hydrolysis of proteins of animal, fungal, or preferably plant origin, which frees peptide fragments containing at least the desired sequence. For example, the compounds of the present invention can be produced by using nucleic acid sequences encoding the amino acid sequences of the polypeptides described herein and optionally performing appropriate amino acid modifications. Merely by way of example, a method for obtaining the polypeptide compounds of the present invention, their stereoisomers and mixtures thereof may comprise the following stages:

- coupling an amino acid with the N-terminus protected and the C-terminus free with an amino acid with the N-terminus free and the C-terminus protected or bound to a solid support;
- eliminating the group protecting the N-terminus;
- repeating the coupling procedure and eliminating the group protecting the N-terminus until the desired peptide sequence is obtained; and
- eliminating the group protecting the C-terminus or cleaving the solid support.

Preferably, the C-terminus is bound to a solid support and the process is carried out in solid phase and therefore comprises coupling an amino acid with the N-terminus protected and the C-terminus free with an amino acid with the N-terminus free and the C-terminus bound to a polymeric support; eliminating the group protecting the N-terminus; and repeating the procedure as many times as is necessary to obtain the compound of the desired length and finally cleaving the synthesized compound from the original polymeric support.

The functional groups of the side chains of the amino acids are maintained conveniently protected with temporary or permanent protective groups throughout synthesis, and can be deprotected simultaneously or orthogonally to the process of cleaving the peptide from the polymeric support.

Alternatively, solid phase synthesis can be carried out using a convergent strategy: coupling a peptide with a polymeric support or with a peptide or an amino acid previously bound to the polymeric support. Convergent synthesis strategies are widely known by those skilled in the art and are described in Lloyd-Williams P. et al., "Convergent Solid-Phase Peptide Synthesis", (1993), Tetrahedron, 49(48), 11065-11133.

The process of the present invention can comprise the additional stages of deprotecting the C-terminus and/or cleaving the peptide from the polymeric support in an indiscriminate order, using standard procedures and conditions known in the prior art, after which the functional groups of these termini can be modified. When the polypeptide compound of formula (I) is fixed to the polymeric support or once the polypeptide compound has been separated from the polymeric support, the optional modification of the C-terminus can be carried out.

Optionally and/or additionally, the $R_1$ residue can be introduced by reacting the compound $HR_1$, wherein $R_1$ is $-OR_2$, $-NR_2R_3$ or $-SR_2$, with a complementary fragment corresponding to the compound of formula (I) in the presence of a suitable solvent and a base such as N,N-diisopropylethylamine (DIEA) or triethylamine or an additive such as N-hydroxybenzotriazole (HOBt) or 1-hydroxyazabenzotriazole (HOAt) and a dehydrating agent such as carbodiimide, a uronium salt, a phosphonium salt or an amidinium salt, wherein $R_1$ is $-NH_2$; or by first allowing a complementary fragment corresponding to the compound of formula (I) and, for example, thionyl chloride to form an acyl halide in advance and then reacting with $HR_1$ to obtain the peptide according to the present invention of general formula (I), wherein the fragment that has the functional groups not involved in the N-C bond formation is suitably protected with temporary or permanent protective groups; or alternatively other $R_1$ residues may be introduced by simultaneous incorporation into the process of cleaving the peptide from the polymeric support.

Those skilled in the art will easily understand that the deprotection/cleavage steps of the C-terminus and the N-terminus and their subsequent derivatization can be performed in a different order according to the processes known in the prior art. The present invention provides a composition comprising the polypeptide compound described above, and an acceptable auxiliary agent.

In the present invention, the composition described above may be a pharmaceutical composition or a health product composition.

In the present invention, the auxiliary agent includes, but is not limited to, carriers, diluents, excipients or auxiliary agents, among others, which are well known to those skilled in the art.

In the present invention, the carriers preferably include, but are not limited to, sterile water, saline, buffers, phosphate-buffered saline, buffered sodium chloride, plant salts, minimal essential medium (MEM), MEM with HEPES,

among others. In the composition of the present invention described above, the polypeptide compound may be present alone, or two or more are present as a mixture, or are more closely associated by complexation, crystallization, or ionic bonding or covalent bonding.

The size of the rigid structure or flexible structure in the C-terminal amino acid residues of the polypeptide compound provided by the present invention is very important for maintaining the configuration of peptide bonds in the sequence, and therefore, different structural types of functional groups are introduced at the C-terminus of the sequence in the present invention, so that the polypeptide compound can effectively bind to GHSR-la and is suitable for treating, preventing, alleviating or diagnosing a related disease caused by a disorder mediated by GHSR-la.

On the basis of this, the present invention provides use of the polypeptide compound described above, or a polypeptide compound prepared using the preparation method described above or the composition described above as an agonist for growth hormone secretagogue receptor, or for preparing a medicament for treating, preventing, alleviating and/or diagnosing a related disease caused by a disorder mediated by growth hormone secretagogue receptor, or as a health product for promoting growth and development.

In the present invention, the growth hormone secretagogue receptor may also be referred to as ghrelin receptor, growth hormone releasing peptide receptor or GHSR-la receptor.

In the present invention, the related disease caused by the disorder mediated by growth hormone secretagogue receptor is preferably growth hormone deficiency. Specifically, the present invention provides the polypeptide compound described above, or a polypeptide compound prepared using the preparation method described above, or use of the composition described above as a GHSR-la agonist. Specifically, the present invention provides the polypeptide compound described above, or a polypeptide compound prepared using the preparation method described above, or use of the composition described above for preparing a GHSR-la agonist.

The above polypeptide compound, composition or agonist for growth hormone secretagogue receptor provided by the present invention may be administered in a variety of ways depending upon whether local or systemic administration is desired and upon the area to be treated. In some embodiments, the polypeptide compound or the composition thereof or the GHSR-la agonist thereof can be administered to the patient orally or rectally, or transmucosally, or intestinally, or intramuscularly, or subcutaneously, or intramedullary, or intrathecally, or direct-intraventricularly, or intravenously, or intravitreally, or intraperitoneally, or intranasally, or intraocularly.

In the present invention, the term "protective group" relates to a group which blocks an organic functional group and which can be removed in controlled conditions. The protective groups, their relative reactivities and the conditions in which they remain inert are known to those skilled in the art.

Examples of representative protective groups for the amino group are particularly amide acetate, amide benzoate, amide pivalate; carbamates such as benzyloxycarbonyl (Cbz or Z), 2-chlorobenzyl (CIZ), p-nitrobenzyloxycarbonyl (pNZ), tert-butyloxycarbonyl (Boc), 2,2,2-trichioroethyloxycarbonyl (Troc), 2-(trimethylsilyl)ethyloxycarbonyl (Teoc), 9-fluorenylmethyloxycarbonyl (Fmoc) or allyloxycarbonyl (Alloc), trityl (Trt), methoxytrityl (Mtt), 2,4-dinitrophenyl (Dnp), N-1-(4,4-dimethyl-2,6-dioxocyclohex-1-ylidene)ethyl (Dde), 1-(4,4-dimethyl-2,6-dioxo-cyclohexylidene)-3-methylbutyl (ivDde), and 1-(1-adamantyl)-1-methylethoxycarbonyl (Adpoc), preferably Boc or Fmoc. Examples of representative protective groups for the carboxyl group are esters, such as tert-butyl ester (tBu), allyl ester (All), triphenylmethyl ester (Trt tester), cyclohexyl ester (cHx), benzyl ester (Bzl), o-nitrobenzyl ester, p-nitrobenzyl ester, p-methoxybenzyl ester, trimethylsilylethyl ester, 2-phenylisopropyl ester, fluorenylmethyl ester (Fm), and 4-(N-[1-(4,4-dimethyl-2,6-dioxo-cyclohexylidene)-3-methylbutyl]amino)benzyl ester (Dmab), preferably All, tBu, cHx, Bzl and Trt esters.

The side chains of the trifunctional amino acids can be protected during synthesis with temporary or permanent protective groups orthogonal to the protective groups of the N-terminus and the C-terminus.

The indole group of the tryptophan side chain can be protected by the formyl group (For), Boc, Mts or can be used unprotected. The piperidinyl group of the 4-amino-4-piperidinecarboxylic acid side chain is protected by Boc or Fmoc. The arginine side chain can be protected by the following protective groups: Tos, 4-methoxy-2,3,6-trimethylbenzenesulfonyl (Mtr), Alloc, nitro, 2,2,4,6,7-pentamethyldihydrobenzofuran-5-sulfonyl (Pbf) and 2,2,5,7,8-pentamethylchroman-6-sulfonyl (Pmc). To protect the amino groups of the lysine and ornithine side chains, amides can be used, such as amide acetate, amide benzoate and amide pivalate; carbamates such as Cbz or Z, CIZ, pNZ, Boc, Troc, Teoc, Fmoc or Alloc, Trt, Mtt, Dnp, Dde, ivDde and Adpoc.

In a preferred embodiment, the protective group strategy used is a strategy in which the amino groups are protected by Boc, the carboxyl groups are protected by Bzl, cHx or All, the arginine side chains are protected by Tos, the piperidinyl groups of the 4-amino-4-piperidinecarboxylic acid side chains are protected by Fmoc, the tryptophan side chains are protected by For or Mts, and the Apc lysine and ornithine side chains are protected by CIZ, Fmoc or Alloc.

In yet another preferred embodiment, the protective group strategy used is a strategy in which the amino groups are protected by Fmoc, the carboxyl groups are protected by tBu, All or Trt esters, the arginine side chains are protected by Pmc or Pbf, the piperidinyl groups of the 4-amino-4-piperidinecarboxylic acid side chains are protected by Boc, the tryptophan side chains are protected by Boc or used unprotected, and the lysine and ornithine side chains are protected by Boc, Trt or Alloc.

Examples of these and other protective groups, their introduction and removal can be found in the literature [Atherton B. and Sheppard R. C., "Solid Phase Peptide Synthesis: A practical approach", (1989), IRL Oxford University Press]. The term "protective group" also includes the polymeric supports used in solid phase synthesis.

Where synthesis takes place fully or partially in solid phase, the possible solid supports used in the process of the present invention involve polystyrene support, polyethylene glycol grafted to polystyrene, and the like, for example and not limited to, p-methylbenzhydrylamine resins (MBHA) [Matsueda G. R. et al., "A p-methyl benzhydrylamine resin for improved solid-phase synthesis of peptide amides", (1981), Peptides, 2, 4550], 2-chlorotrityl resins [Barlos K. et al., "Darstellung geschützter PeptidFragmente unter Einsatz substituierter Triphenylmethyl Harze", (1989), Tetrahedron Lett., 30, 3943-3946; Barlos K. et al., "Veresterung von partiell geschützten PeptidFragmenten mit Harzen Einsatz von 2-Chlorotritylchlorid zur Synthese von LeulGastrin I", (1989), Tetrahedron Lett., 30, 39473951], TentaGel® resins (Rapp Polymere GmbH), ChemMatrix® resins (Matrix Innovation, Inc), and the like, which may or may not include a labile linker, such as 5-(4-aminomethyl-3,5-dimethoxyphenoxy)valeric acid (PAL) [Albericio F. et al., "Preparation and application of the 5-(4-(9-fluorenylmethyloxycarbonyl)aminomethyl-3,5-dimethoxy-phenoxy)valeric acid (PAL) handle for the solid-phase synthesis of C-terminal peptide amides under mild conditions", (1990), J. Org. Chem., 55, 3730-3743], 2-[4-aminomethyl-(2,4-dimethoxyphenyl)]phenoxyacetic acid (AM) [Rink H., "Solid-phase synthesis of protected peptide fragments using a trialkoxy-diphenyl-methylester resin", (1987), Tetrahedron Lett., 28, 3787-3790], Wang [Wang S.S., "p-Alkoxybenzyl Alcohol Resin and p- Alkoxybenzyl oxycarbonylhydrazide Resin for Solid Phase Synthesis of Protected Peptide Fragments", (1973), J. Am. Chem. Soc, 95,1328-1333], and the like, which enable simultaneous deprotection and cleavage of the peptide from the polymeric support.

Definitions

The abbreviations used in the present invention have the following meanings:

Ala (Alanine, A)
Aba (2-aminobutyric acid)
Apc (4-Amino-4-piperidine formic acid)
Arg (Arginine, R)
Bal (3-Benzothienylalanine)
Boc (butyloxycarboryl)
Cit (Citrulline)
DCM (Dichloromethane)
DIEA (N,N-Diisopropylethylamine)
DMF (N,N-Dimethylformamide)
Fmoc (Fluorenylmethoxycarbonyl)
Gly (Glycine, G)
HBTU (O-Benzotriazole-N,N,N',N'-tetraMethyl-uroniuM-hexafluorophosphate)
HOBt (N-Hydroxybenzotrizole)
HPLC (High performance liquid chromatography)
Leu (Leucine, L)
Lys (Lysine, K)
Nle (Norleucine)
Orn (Ornithine)
Phe (3-Amino-4-phenylbutyric acid, F)
Pro (Proline, P)
TFA (Trifluoroacetic acid)
Tle (Tertiary leucine)
Trp (Tryptophan, W)
Val (Valine, V)

As defined herein, the terms "polypeptide", "peptide" and "amino acid sequence" are used interchangeably herein to refer to a polymer of amino acid residues of any length. The polymer may be linear or branched, it may comprise modified amino acids or amino acid analogs, and it may be interrupted by chemical moieties other than amino acids. The terms also encompass amino acid polymers which have been modified naturally or artificially (for example, disulfide bond formation, glycosylation, lipidation, acetylation, phosphorylation or any other manipulation or modification, such as conjugation with a labeling or bioactive component). The term "peptide" encompasses two or more naturally-occurring or synthetic amino acids linked by covalent bonds (for example, amido bonds).

In the context of the present disclosure, the term "amino acid" is defined as having at least one primary,

secondary, tertiary or quaternary amino group and at least one acid group, wherein the acid group may be a carboxylic acid, sulfonic acid or phosphoric acid or a mixture thereof. The amino groups may be "$\alpha$", "$\beta$", "$\gamma$" to "$\omega$" with respect to the acid group. Suitable amino acids include, but are not limited to, the D- and L-isomers of the 20 common naturally-occurring amino acids found in peptides (e.g., alanine, arginine, asparagine, cysteine, glutamine, aspartic acid, glutamic acid, glycine, histidine, isoleucine, leucine, lysine, methionine, phenylalanine, proline, serine, threonine, tryptophan, tyrosine and valine), and the naturally-occurring and non-naturally-occurring amino acids prepared by organic synthesis or other metabolic routes.

"The backbone of an amino acid" may be substituted with one or more groups selected from halogen, hydroxyl, guanidino and heterocyclic groups. Therefore, the term "amino acid" also encompasses within its scope glycine, alanine, valine, leucine, isoleucine, norleucine, methionine, proline, phenylalanine, tryptophan, serine, threonine, cysteine, tyrosine, asparagine, glutamine, aspartic acid, glutamic acid, lysine, histidine, homocysteine, taurine, betaine, N-methylalanine, and the like. (L)- and (D)-amino acids are encompassed.

The term "amino acid side chain" refers to the moiety attached to the $\alpha$-carbon of an amino acid. For example, the amino acid side chain of alanine is methyl, the amino acid side chain of phenylalanine is phenylmethyl, the amino acid side chain of cysteine is thiomethyl, the amino acid side chain of aspartate is carboxymethyl, the amino acid side chain of tyrosine is 4-hydroxyphenylmethyl, etc. Other non-naturally-occurring amino acid side chains are also included, for example, those that occur naturally (e.g., amino acid metabolites) or those that are prepared synthetically (e.g., $\alpha$-substituted amino acids).

As used herein, the term "acyclic aliphatic group" encompasses linear or branched alkyl, alkenyl and alkynyl groups.

The term "alkyl" refers to a linear or branched saturated group which has 1 to 24, preferably 1 to 16, more preferably 1 to 14, even more preferably 1 to 12, and yet more preferably 1, 2, 3, 4, 5 or 6 carbon atoms and is bound to the rest of the molecule by a simple bond, including, for example and not limited to, methyl, ethyl, isopropyl, isobutyl, tert-butyl, heptyl, octyl, decyl, dodecyl, lauryl, hexadecyl, octadecyl, pentyl, 2-ethylhexyl, 2-methylbutyl, 5-methylhexyl, and the like.

The term "alkenyl group" refers to a linear or branched group which has 2 to 24, preferably 2 to 16, more preferably 2 to 14, even more preferably 2 to 12, and yet more preferably 2, 3, 4, 5 or 6 carbon atoms and one or more, preferably 1, 2 or 3, conjugated or unconjugated carbon-carbon double bonds, which is bound to the rest of the molecule by a simple bond, including, for example and not limited to, vinyl ($-CH_2=CH_2$), allyl ($-CH_2-CH=CH_2$), oleyl, linoleyl, and the like.

The term "alkynyl group" refers to a linear or branched group which has 2 to 24, preferably 2 to 16, more preferably 2 to 14, even more preferably 2 to 12, and yet more preferably 2, 3, 4, 5 or 6 carbon atoms and one or more, preferably 1, 2 or 3, conjugated or unconjugated carbon-carbon triple bonds, which is bound to the rest of the molecule by a simple bond, including, for example and not limited to, the ethynyl group, 1-propynyl, 2-propynyl, butynyl such as 1-butynyl, 2-butynyl or 3-butynyl, pentynyl such as 1-pentynyl, and the like. Alkynyl groups may also contain one or more carbon-carbon double bonds, including, for example and not limited to, the group but-1-en-3-ynyl, pent-4-en-1-ynyl, and the like.

The term "alicyclic group" is used in the present invention to encompass, for example and not limited to, cycloalkyl or cycloalkenyl or cycloalkynyl groups.

The term "cycloalkyl" refers to a saturated, mono- or polycyclic aliphatic group which has 3 to 24, preferably 3 to 16, more preferably 3 to 14, even more preferably 3 to 12, and yet more preferably 3, 4, 5 or 6 carbon atoms and is bound to the rest of the molecule by a simple bond, including, for example and not limited to, cyclopropyl, cyclobutyl, cyclopentyl, cyclohexyl, cycloheptyl, methylcyclohexyl, dimethylcyclohexyl, octahydroindene, decahydronaphthalene, dodecahydrophenalene, and the like.

The term "cycloalkenyl" refers to a non-aromatic, mono- or polycyclic aliphatic group which has 5 to 24, preferably 5 to 16, more preferably 5 to 14, even more preferably 5 to 12, and yet more preferably 5 or 6 carbon atoms and one or more, preferably 1, 2 or 3, conjugated or unconjugated carbon-carbon double bonds, which is bound to the rest of the molecule by a simple bond, including, for example and not limited to, the cyclopent-1-en-1-yl group, and the like.

The term "cycloalkynyl" refers to a non-aromatic, mono- or polycyclic aliphatic group which has 8 to 24, preferably 8 to 16, more preferably 8 to 14, even more preferably 8 to 12, and yet more preferably 8 or 9 carbon atoms and one or more, preferably 1, 2 or 3, conjugated or unconjugated carbon-carbon triple bonds, which is bound to the rest of the molecule by a simple bond, including, for example and not limited to, the cyclooct-2-yn-1-yl group, and the like. Cycloalkynyl groups may also contain one or more carbon-carbon double bonds, including, for example and not limited to, the cyclooct-4-en-2-ynyl group, and the like.

The term "aryl group" refers to an aromatic group which has 6 to 30, preferably 6 to 18, more preferably 6 to 10, and even more preferably 6 or 10 carbon atoms and comprises 1, 2, 3 or 4 aromatic rings bound by a carbon-carbon bond or fused, including, for example and not limited to, phenyl, naphthyl, diphenyl, indenyl, phenanthryl or anthranyl, among others; or an aralkyl group.

The term "aralkyl group" refers to an alkyl group substituted with an aromatic group and having 7 to 24 carbon atoms, including, for example and not limited to, $-(CH_2)_{1-6}$-phenyl, $-(CH_2)_{1-6}$-(1-naphthyl), $-(CH_2)_{1-6}$-(2-naphthyl), $-(CH_2)_{1-6}$-CH(phenyl)$_2$, and the like.

The term "heterocyclyl group" refers to a 3-10 membered hydrocarbonated ring in which one or more of the atoms in the ring, preferably 1, 2 or 3 of the atoms in the ring, are different elements from carbon, such as nitrogen, oxygen or sulfur, and can be saturated or unsaturated. For the purposes of the present invention, the heterocycle can be a monocyclic, bicyclic or tricyclic system, which may include fused ring systems; and the nitrogen, carbon or sulfur atoms in the residual heterocycle can optionally be oxidized; the nitrogen atom can optionally be quaternized; and the residual heterocyclyl can be partially or completely saturated or aromatic. The term heterocyclyl most preferably refers to a 5- or 6-membered ring. Examples of saturated heterocyclyl groups are dioxane, piperidine, piperazine, pyrrolidine, morpholine and thiomorpholine. Examples of aromatic heterocyclyl groups, also known as heteroaromatic groups, are pyridine, pyrrole, furan, thiophene, benzofuran, imidazoline, hydroquinone, quinoline and naphthyridine. The term "heteroarylalkyl group" refers to an alkyl group substituted with a substituted or unsubstituted aromatic heterocyclyl group, the alkyl group having 1 to 6 carbon atoms and the aromatic heterocyclyl group having 2 to 24 carbon atoms and 1 to 3 atoms other than carbon and including, for example and not limited to, $-(CH_2)_{1-6}$-imidazolyl, $-(CH_2)_{1-6}$-triazolyl, $-(CH_2)_{1-6}$-thienyl, $-(CH_2)_{1-6}$-furyl, $-(CH_2)_{1-6}$-pyrrolidinyl, and the like.

The term "halogen" or variants such as "halide" or "halo" as used herein refers to fluorine, chlorine, bromine and iodine.

The term "heteroatom" or variants such as "hetero-" as used herein refers to O, N, NH and S.

The term "alkoxy" as used herein refers to a linear or branched alkoxy group. Examples include methoxy, ethoxy, n-propoxy, isopropoxy, tert-butoxy, and the like.

The term "amino" as used herein refers to groups of the form $-NR_aR_b$, wherein $R_a$ and $R_b$ are independently selected from the group including but not limited to hydrogen, optionally substituted alkyl, optionally substituted alkenyl, optionally substituted alkynyl and optionally substituted aryl.

It will be appreciated that the compounds described herein may be substituted with any number of substituents or functional moieties. In general, the term "substituted", whether preceded by the term "optionally" or not, and the substituents contained in the formula of the present invention, refer to the replacement of hydrogen radicals in a given structure with the radical of a specified substituent. When more than one position in any given structure may be substituted with more than one substituent selected from a specified group, the substituent may be the same or different at each position. The term "substituted" as used herein is contemplated to include substitution with all permissible substituents of organic compounds and any of the substituents described herein.

For example, the substituents include, but are not limited to, the following groups that result in the formation of a stable moiety: aliphatic groups, alkyl, alkenyl, alkynyl, heteroaliphatic groups, heterocyclyl, aryl, heteroaryl, acyl, oxo, imino, thiooxo, cyano, isocyano, amino, azido, nitro, hydroxyl, thiol and halo and any combination thereof including, but not limited to, the following groups: aliphaticamino, heteroaliphaticamino, alkylamino, heteroalkylamino, arylamino, heteroarylamino, alkylaryl, arylalkyl, aliphaticoxy, heteroaliphaticoxy, alkoxy, heteroalkoxy, aryloxy, heteroaryloxy, aliphaticthio, heteroaliphaticthio, alkylthio, heteroalkylthio, arylthio, heteroarylthio, acyloxy, and the like. The present invention encompasses any and all such combinations in order to obtain a stable substituent/moiety. For purposes of the present invention, heteroatoms such as nitrogen may have hydrogen substituents and/or any suitable substituent as described herein which satisfies the valences of the heteroatoms and results in the formation of a stable moiety.

The compounds may contain one or more asymmetric centers and therefore exist as racemates and racemic mixtures, single enantiomers, individual diastereomers and mixtures of diastereomers. All such isomeric forms of these compounds are expressly included herein. The compounds may also be represented in a variety of tautomeric forms; in such cases, all tautomeric forms of the compounds described herein are expressly included herein (for example, alkylation of a ring system may lead to alkylation at multiple sites; all such reaction products are expressly included herein). All such isomeric forms of such compounds are expressly included herein. All crystal forms of the compounds described herein are expressly included herein. The compounds of the present invention can exist as stereoisomers or mixtures of stereoisomers; for example, the amino acids which constitute them can have the configuration L-, D-, or be racemic, independently of each other. Therefore, it is possible to obtain isomeric mixtures as well as racemic mixtures or diastereomeric mixtures, or pure diastereomers or enantiomers, depending upon the number of asymmetric carbons and upon the asymmetric carbons present in isomers or isomeric mixtures. The preferred structures of the compounds of the present invention are pure isomers, i.e., enantiomers or diastereomers.

For example, when it is stated that $U_2$ can be -Lys-, it is understood that $U_2$ is selected from -L-Lys-, -D-Lys- and mixtures of both and is racemic or non-racemic. The preparation process described in this document enables those skilled in the art to obtain each of the stereoisomers of the compounds of the present invention by selecting amino acids with the right configurations.

Pharmaceutically acceptable salts of the peptide of the present invention also fall within the scope of the present invention. The term "pharmaceutically acceptable salt" means a salt whose use in animals and more specifically in

humans is recognized, and encompasses salts used to form base addition salts, they being either inorganic salts, such as and not limited to, lithium, sodium, potassium, calcium, magnesium, manganese, copper, zinc or aluminum, among others, or organic salts, such as and not limited to, ethylamine, diethylamine, ethanolamine, diethanolamine, arginine, lysine, histidine or piperazine, among others; or acid addition salts, they being either organic salts, such as and not limited to, acetate, citrate, lactate, malonate, maleate, tartrate, fumarate, benzoate, aspartate, glutamate, succinate, oleate, trifluoroacetate, oxalate, pamoate or gluconate, among others, or inorganic salts, such as and not limited to, hydrochloride, sulfate, phosphate, borate or carbonate, among others. The nature of the salts is not critical, provided that it is cosmetically or pharmaceutically acceptable. The pharmaceutically acceptable salts of the peptide of the present invention can be obtained using conventional methods well known in the prior art (Berge S. M. et al., "Pharmaceutical Salts", (1977), J. Pharm. Sci., 66, 119, which is incorporated herein by reference in its entirety). Compared to the prior art, the present invention provides a polypeptide compound having a structure represented by formula I, or a stereoisomer, mixture or pharmaceutically acceptable salt thereof. Experimental results show that the polypeptide compound provided by the present invention can effectively exhibit high agonistic activity for GHSR-la.

## DETAILED DESCRIPTION

To further illustrate the present invention, the polypeptide compound provided by the present invention and use thereof are described in detail below using examples. Those skilled in the art will appreciate that the following examples are only for illustrating the present invention, and should not be construed as limitations to the scope of the present invention. Experimental procedures without specified conditions in the examples are conducted according to conventional conditions or conditions recommended by the manufacturer. Reagents or instruments used herein without specified manufacturers are conventional products that are commercially available.

The polypeptide was synthesized using a standard Fmoc solid phase method. Rink Amide resin was selected. The peptide chain extends from the C-terminus to the N-terminus. Protected amino acids include: Fmoc-Apc(Boc)-OH, Fmoc-D-Lys(Boc)-OH, Fmoc-D-Orn(Boc)-OH, Fmoc-Phe-OH, Fmoc-D-Trp(Boc)-OH, Fmoc-D-Bal-OH, Fmoc-D-Cit-OH, Fmoc-D-Arg(Pbf)-OH, Boc-D-Aba-OH, Fmoc-Lys(Boc)-OH, Fmoc-Lys(Alloc)-OH, Fmoc-Gly-OH, Fmoc-D-Ala-OH, Fmoc-D-Val-OH, Fmoc-D-Leu-OH, Fmoc-Pro-OH, Fmoc-β-Ala-OH, Fmoc-D-Tle-OH, Fmoc-Tle-OH, Fmoc-D-Nle-OH, Fmoc-Nle-OH, cis-2-(tert-butoxycarbonylamide)-1-cyclopentanecarboxylic acid, Boc-methyl-1-(aminomethyl)cyclobutanecarboxylic acid, 1-N-Boc-3-azetidinecarboxylic acid, Boc-3-aminooxetane-3-carboxylic acid, 1-Boc-D-acridine-2-carboxylic acid, (S)-1-Boc-pyrrolidine-3-carboxylic acid, Boc-2-morpholinecarboxylic acid, (Boc-3-amino-1-adamantane)acetic acid, (1R,3S,4S)-N-Boc-2-azabicyclo[2.2.1]heptane-3-carboxylic acid, and 3-Boc-3-azabicyclo[3.1.0]hexane-1-carboxylic acid. The condensing agent was HBTU/HOBt/DIEA. The deprotecting reagent was piperidine/DMF solution. The crude peptide was dissolved in water and then lyophilized and stored. Separation and purification were carried out by medium-pressure liquid chromatography or high performance liquid chromatography (HPLC). The pure peptide content was greater than 90%. The molecular weight of the peptide sequence was determined by matrix-assisted laser desorption/ionization time-of-flight mass spectrometry (MALDI-TOF-MS).

Synthesis of the peptide sequence:
The synthesis conditions were as follows:

protected amino acids (natural or non-natural): 0.2 M solutions in DMF;
condensing agent: a 0.45 M solution of HBTU/HOBt in DMF;
activating base: a 2 M solution of DIEA in DMF;
deprotecting reagent: a 20% v/v solution of piperidine in DMF.

Example 1

Preparation of Compounds 1-5, 9, 11-35, 39 and 41-80:

1. Deprotection: 0.23 g (0.1 mmol) of Rink Amide resin was weighed out and placed into a polypeptide synthesis reactor. Then the deprotecting reagent was prepared according to the concentration described above and added to the resin. The mixture was allowed to react at room temperature. The resin was dried under vacuum, and piperidine/DMF was added again. The mixture was allowed to react at room temperature, and then the resin was dried under vacuum and washed with DMF until tests showed it was acceptable.
2. Condensation reaction: Amino acids and the condensing agent were added to DMF for activation in an ice bath, and the activating base was added for reaction to obtain an activating solution. The activating solution was finally added to the resin. After reaction at room temperature, the resin was colored with 5% ninhydrin color-developing reagent; the color of the resin changed. The solvent was removed under vacuum and the resin was washed with DMF. After tests showed the resin was acceptable, the solvent was removed under vacuum, and the condensation

reaction was complete by now.

3. The deprotection and condensation reaction described above was repeated until peptide chain synthesis was complete; a peptide resin containing a complete polypeptide sequence structure was obtained.

4. Cleavage of the peptide resin: 1.25 g of the synthesized peptide resin was weighed out, placed into a 250 mL eggplant-shaped flask, cooled in an ice bath and electromagnetically stirred. A cleavage solution was prepared in such an amount that the solution would be added at 10 mL per gram of the peptide resin (cleavage solution (volume percentage):trifluoroacetic acid:thioanisole:water = 90:5:5). TFA should be pre-cooled in an ice bath for 30 min or stored beforehand in a refrigerator before use. The prepared cleavage solution was added to the peptide resin in an ice bath. The mixture was electromagnetically stirred, and the resin turned black. The reaction was performed in the ice bath for 30 min, and then the ice bath was removed. The reaction was stirred at room temperature for another 180 min, and the reaction was complete. 200 mL of cold diethyl ether was added with vigorous stirring, and a white crystal precipitated. The stirring was continued for 30 min. The precipitate was collected by filtration using a G4 sand core funnel, washed 3 times with cold diethyl ether, and allowed to dry. 50 mL of double distilled water and 5 mL of acetonitrile were added to fully dissolve the solid. The solution was filtered under vacuum, and the filtrate was lyophilized to give a crude peptide (1.04 g).

5. Purification of the crude peptide: The crude peptide was purified by medium-pressure or high performance liquid chromatography. The chromatography column was a C18 column. The eluent was acetonitrile, water and a small amount of acetic acid. Specifically, 1.00 g of the crude peptide was weighed out, and 20 mL of water and 5 mL of acetonitrile were added to dissolve the solid; the solution was centrifuged at 5000 rpm for 10 min, and the supernatant was collected for sample injection. The chromatography column was equilibrated beforehand with 200 mL of a solution of 15% acetonitrile/water/0.1% glacial acetic acid. After sample injection, a wash was performed using another 200 mL of a solution of 15% acetonitrile/water/0.1% glacial acetic acid, and the composition of the eluate was determined by high performance liquid chromatography. According to the liquid chromatography results, the acetonitrile content was gradually increased until the main peak of the purified polypeptide was obtained by elution. The eluates were combined and concentrated by rotary evaporation to remove most of the solvent. The purified polypeptide was lyophilized, with a content of greater than 90% as determined by HPLC. The molecular weight was confirmed by MALDI-TOF-MS.

Example 2

Preparation of Compounds 10 and 40:

This example is based on Example 1, and is different from Example 1 in that the last protected amino acid at the N-terminus of the peptide chain is Fmoc-Lys(Alloc)-OH. The method of removing the protective group for its side chain is as follows: triphenylphosphine palladium and phenylsilane (1:10, v/v) were added to the resin that had been dried under vacuum, the mixture was allowed to react under $N_2$ in a dark place for 3 h, detection showed the color of the resin had changed, and deprotection was complete; after the resin was washed and dried under vacuum, an acetylation reaction was performed: 2 mL of acetic acid and 2 mL of DIEA were added, the mixture was allowed to react for 30 min, and the resin was washed and dried under vacuum. Finally, the Fmoc protective group was removed in the 20% v/v solution of piperidine in DMF, and the product was obtained after cleavage and purification.

Example 3

Preparation of Compounds 6-8 and 36-38:

This example is based on Example 1, and is different from Example 1 in that after the deprotection of the last amino acid at the N-terminus of the peptide chain was complete, 2 mL of acetic acid and 2 mL of DIEA were added, the mixture was allowed to react for 30 min, acetylation capping was performed, and finally the product was obtained after cleavage and purification.

The polypeptide compounds prepared using the synthesis methods of the embodiments disclosed herein are shown in Table 1 below.

Table 1. Synthesized polypeptide compounds

| SEQ ID NO. | Structure | Calculated M.W. (g/mol) | ObservedM.W. (g/mol) | Purity (%) |
|---|---|---|---|---|
| 1 | 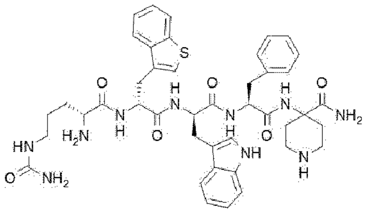 | 837.01 | 838.0 | 90.0 |
| 2 | 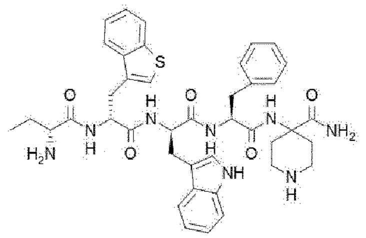 | 764.95 | 765.5 | 92.7 |
| 3 | 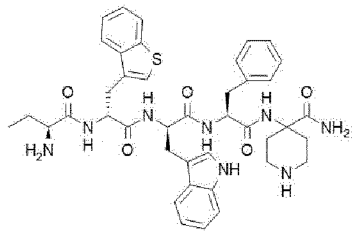 | 764.95 | 765.6 | 91.7 |
| 4 | 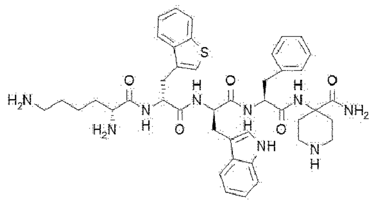 | 808.01 | 808.5 | 93.7 |
| 5 | 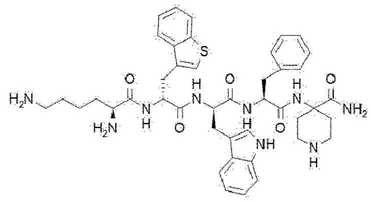 | 808.01 | 808.4 | 91.3 |
| 6 | 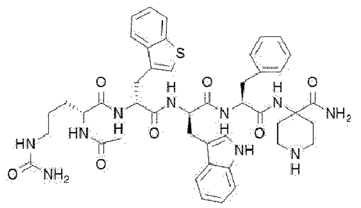 | 879.05 | 879.5 | 98.2 |
| 7 | 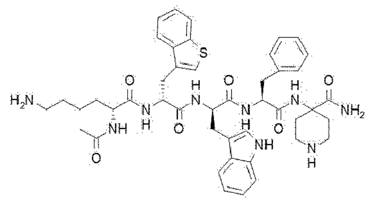 | 850.05 | 850.2 | 98.5 |

(continued)

| SEQ ID NO. | Structure | Calculated M.W. (g/mol) | ObservedM.W. (g/mol) | Purity (%) |
|---|---|---|---|---|
| 8 | 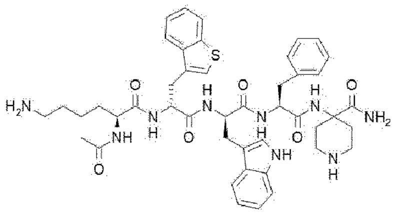 | 850.05 | 850.4 | 96.4 |
| 9 | 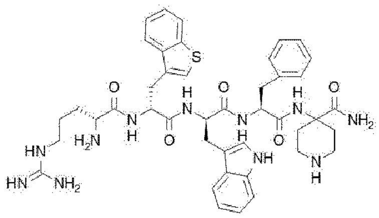 | 836.03 | 837.0 | 93.7 |
| 10 | 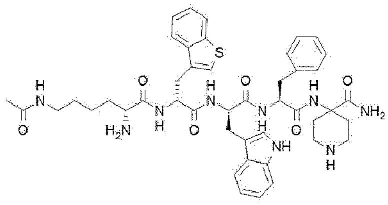 | 850.05 | 850.6 | 95.3 |
| 11 | 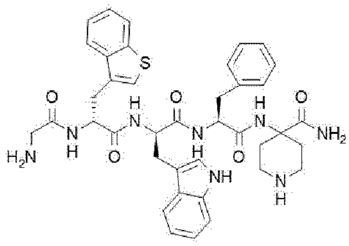 | 736.89 | 737.5 | 97.3 |
| 12 | 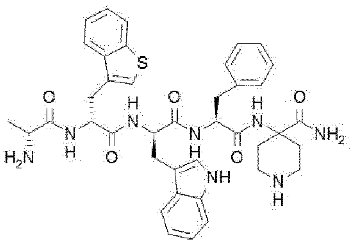 | 750.92 | 751.7 | 93.6 |
| 13 | 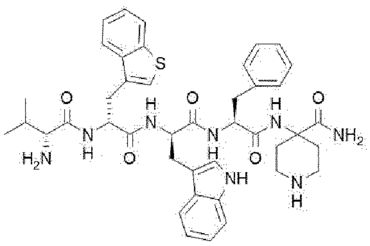 | 778.97 | 779.4 | 95.3 |

(continued)

| SEQ ID NO. | Structure | Calculated M.W. (g/mol) | ObservedM.W. (g/mol) | Purity (%) |
|---|---|---|---|---|
| 14 | 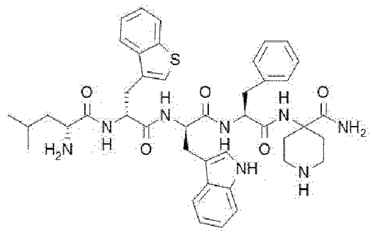 | 793 | 793.7 | 99.8 |
| 15 | 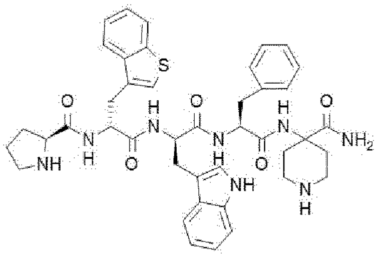 | 776.96 | 777.6 | 94.9 |
| 16 | 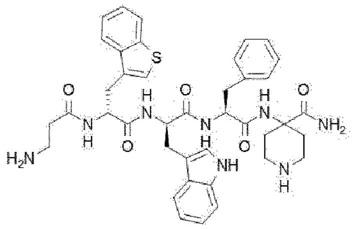 | 750.92 | 751.0 | 97.1 |
| 17 | 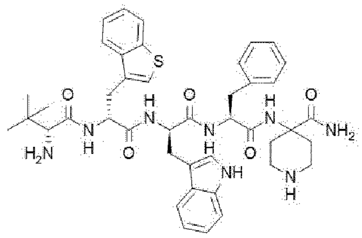 | 793 | 793.5 | 92.1 |
| 18 | 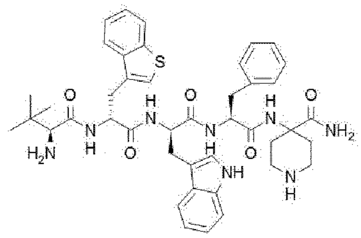 | 793 | 793.3 | 93.6 |
| 19 | 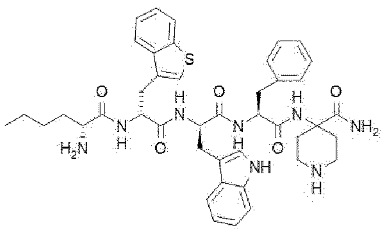 | 793 | 793.7 | 95.2 |

(continued)

| SEQ ID NO. | Structure | Calculated M.W. (g/mol) | ObservedM.W. (g/mol) | Purity (%) |
|---|---|---|---|---|
| 20 | 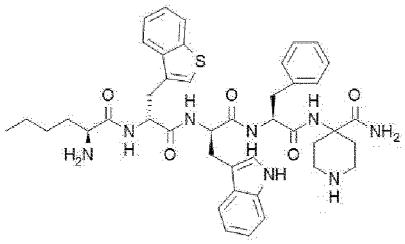 | 793 | 793.9 | 94.9 |
| 21 | 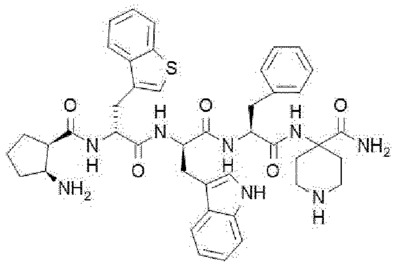 | 790.98 | 791.7 | 97.4 |
| 22 | 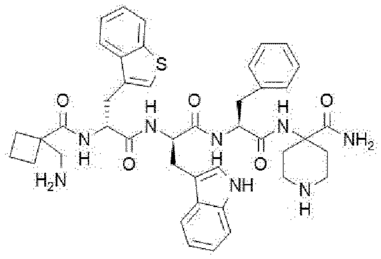 | 790.98 | 791.9 | 97.1 |
| 23 | 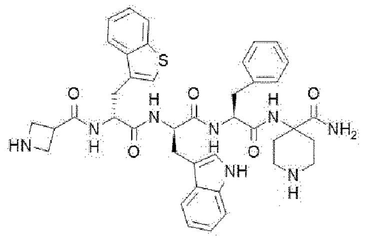 | 762.93 | 763.6 | 94.8 |
| 24 | 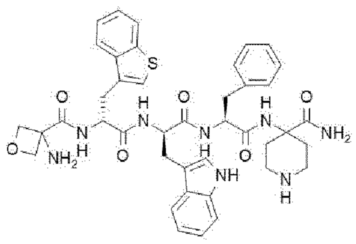 | 778.93 | 779.5 | 90.8 |
| 25 | 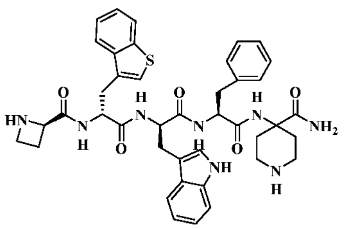 | 762.93 | 763.6 | 93.7 |

(continued)

| SEQ ID NO. | Structure | Calculated M.W. (g/mol) | ObservedM.W. (g/mol) | Purity (%) |
|---|---|---|---|---|
| 26 | 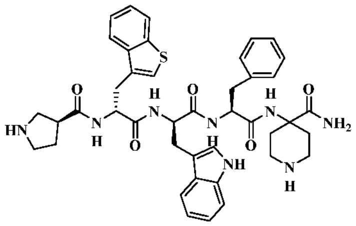 | 776.96 | 777.8 | 93.6 |
| 27 | 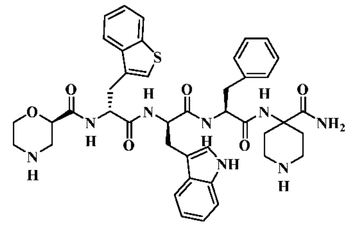 | 792.96 | 793.4 | 95.4 |
| 28 | 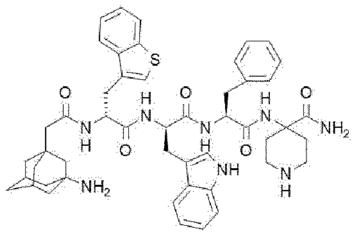 | 871.11 | 872.0 | 96.6 |
| 29 | 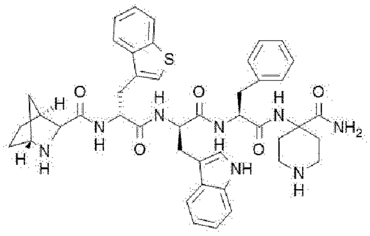 | 802.99 | 803.4 | 92.2 |
| 30 | 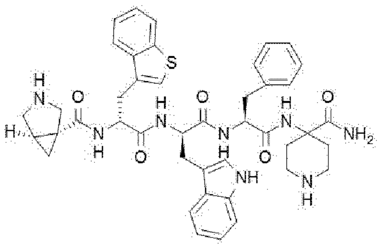 | 788.97 | 789.5 | 93.7 |
| 31 | 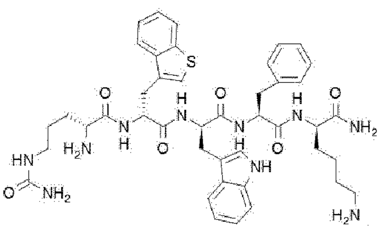 | 839.03 | 839.7 | 94.0 |

(continued)

| SEQ ID NO. | Structure | Calculated M.W. (g/mol) | ObservedM.W. (g/mol) | Purity (%) |
|---|---|---|---|---|
| 32 | 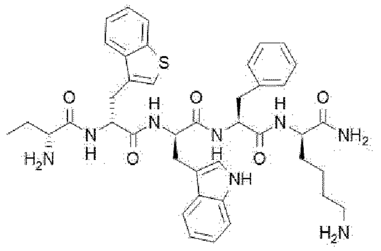 | 766.96 | 767.4 | 95.5 |
| 33 | 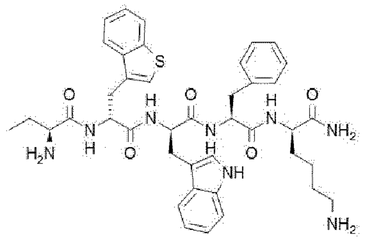 | 766.96 | 767.8 | 91.2 |
| 34 | 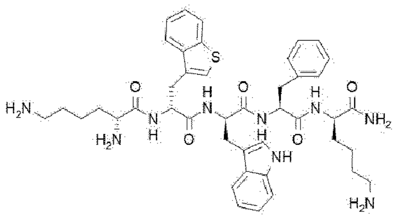 | 810.03 | 810.6 | 92.0 |
| 35 | 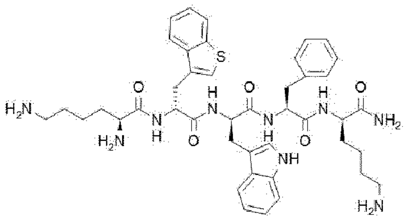 | 810.03 | 810.4 | 93.2 |
| 36 | 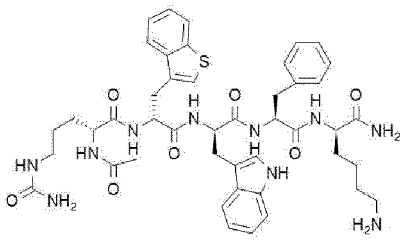 | 881.07 | 882.0 | 92.8 |
| 37 | 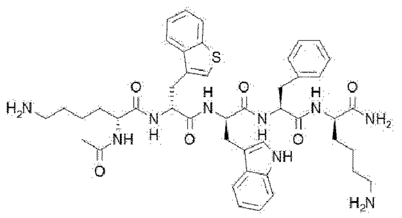 | 852.07 | 852.9 | 97.4 |

(continued)

| SEQ ID NO. | Structure | Calculated M.W. (g/mol) | ObservedM.W. (g/mol) | Purity (%) |
|---|---|---|---|---|
| 38 | 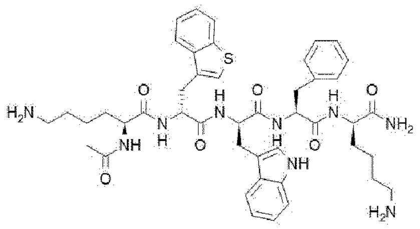 | 852.07 | 852.7 | 93.1 |
| 39 | 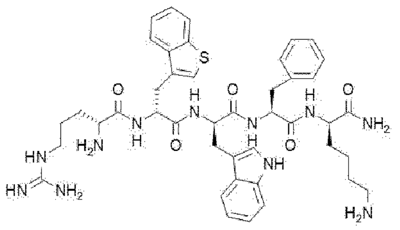 | 838.04 | 838.6 | 93.8 |
| 40 | 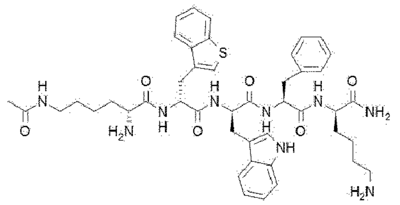 | 852.07 | 852.8 | 92.2 |
| 41 | 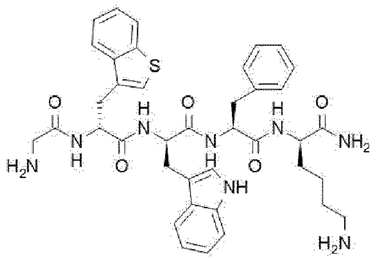 | 738.91 | 739.8 | 92.7 |
| 42 | 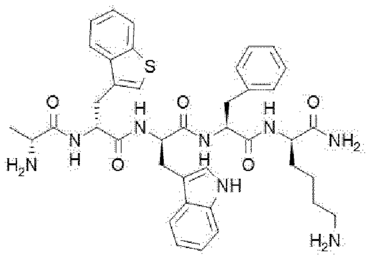 | 752.93 | 753.9 | 93.5 |
| 43 | 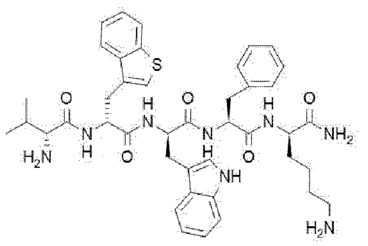 | 780.99 | 781.4 | 94.2 |

(continued)

| SEQ ID NO. | Structure | Calculated M.W. (g/mol) | ObservedM.W. (g/mol) | Purity (%) |
|---|---|---|---|---|
| 44 | 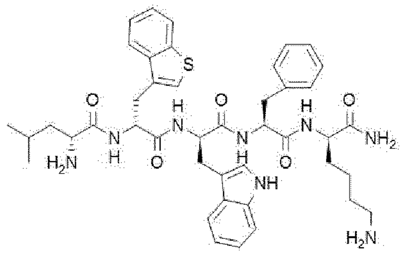 | 795.02 | 796.0 | 96.1 |
| 45 | 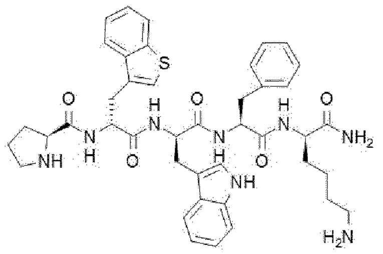 | 778.97 | 779.8 | 95.0 |
| 46 | 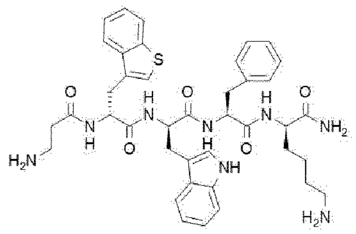 | 752.93 | 753.4 | 92.6 |
| 47 | 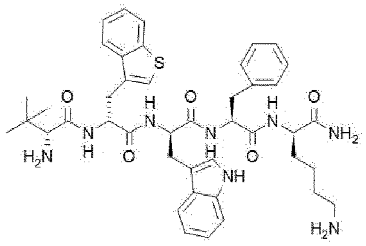 | 795.02 | 795.7 | 93.0 |
| 48 | 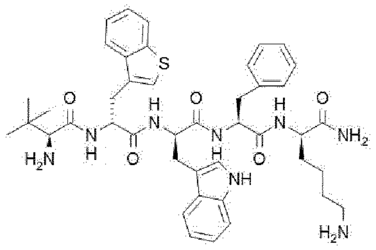 | 795.02 | 795.3 | 94.3 |
| 49 | 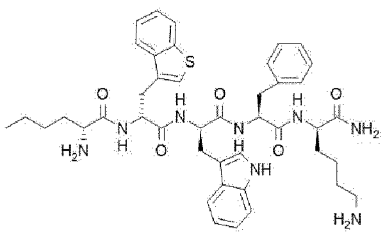 | 795.02 | 795.9 | 92.5 |

(continued)

| SEQ ID NO. | Structure | Calculated M.W. (g/mol) | ObservedM.W. (g/mol) | Purity (%) |
|---|---|---|---|---|
| 50 | 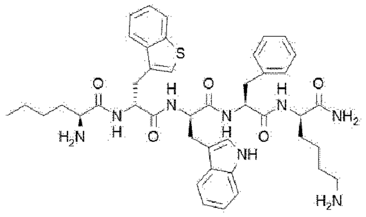 | 795.02 | 796.0 | 90.9 |
| 51 | 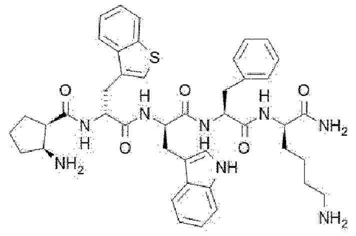 | 793 | 793.7 | 91.4 |
| 52 | 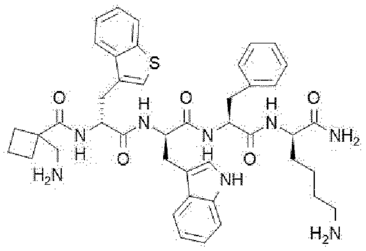 | 793 | 793.6 | 93.8 |
| 53 | 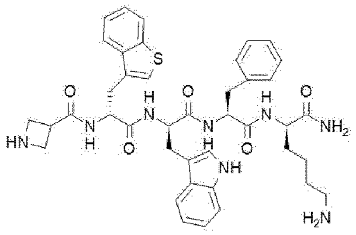 | 764.95 | 765.7 | 92.3 |
| 54 | 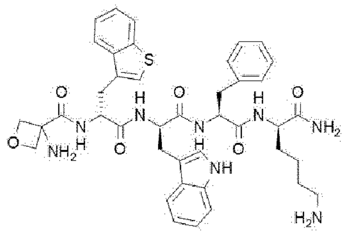 | 780.94 | 781.3 | 92.2 |
| 55 | 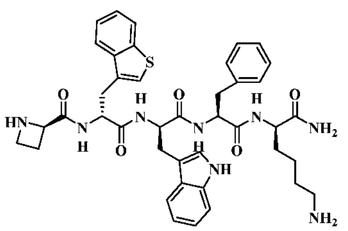 | 764.95 | 765.8 | 92.7 |

(continued)

| SEQ ID NO. | Structure | Calculated M.W. (g/mol) | ObservedM.W. (g/mol) | Purity (%) |
|---|---|---|---|---|
| 56 | 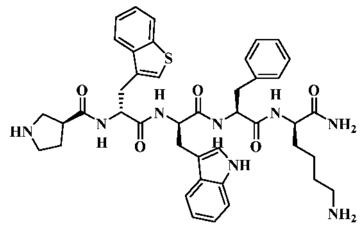 | 778.97 | 779.4 | 93.2 |
| 57 | 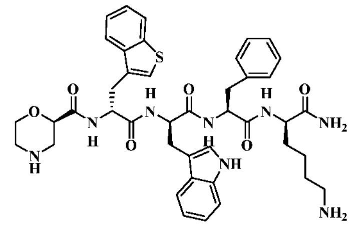 | 794.97 | 795.6 | 92.6 |
| 58 | 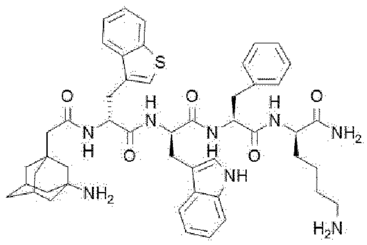 | 873.13 | 874.0 | 96.4 |
| 59 | 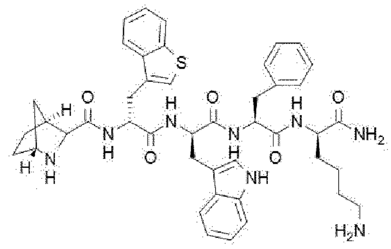 | 805.01 | 805.7 | 93.4 |
| 60 | 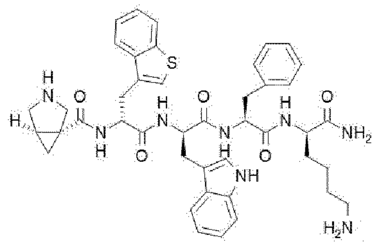 | 790.98 | 791.6 | 92.2 |
| 61 | 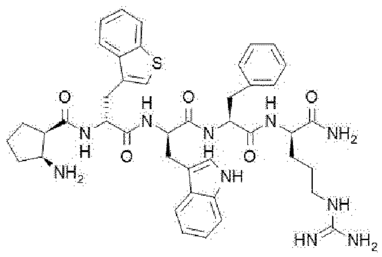 | 821.01 | 821.7 | 93.5 |

(continued)

| SEQ ID NO. | Structure | Calculated M.W. (g/mol) | ObservedM.W. (g/mol) | Purity (%) |
|---|---|---|---|---|
| 62 | 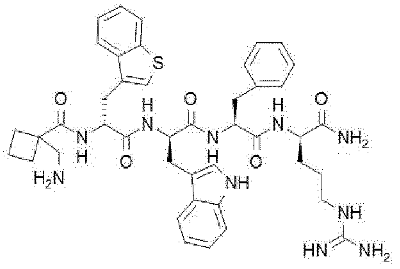 | 821.01 | 821.2 | 92.5 |
| 63 | 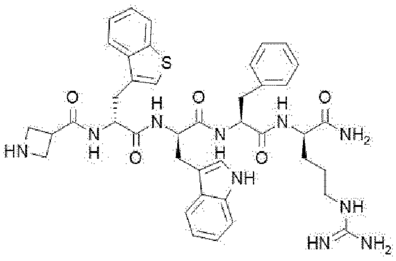 | 792.96 | 793.8 | 92.6 |
| 64 | 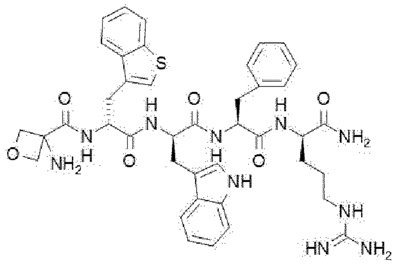 | 808.96 | 809.3 | 95.0 |
| 65 | 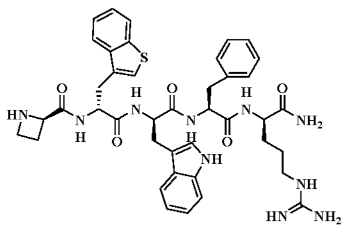 | 792.96 | 793.6 | 94.8 |
| 66 | 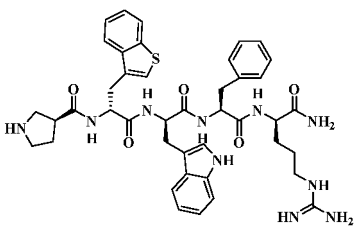 | 806.99 | 807.5 | 93.7 |
| 67 | 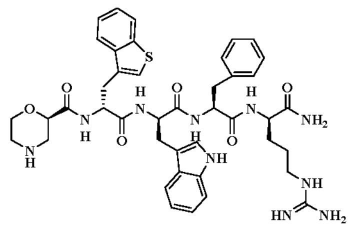 | 822.99 | 823.3 | 95.3 |

(continued)

| SEQ ID NO. | Structure | Calculated M.W. (g/mol) | ObservedM.W. (g/mol) | Purity (%) |
|---|---|---|---|---|
| 68 | 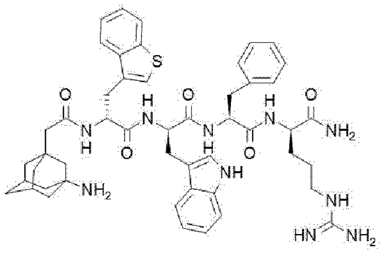 | 901.14 | 901.8 | 94.7 |
| 69 | 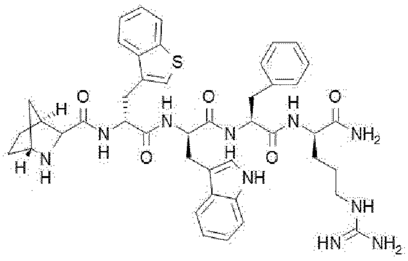 | 833.02 | 833.2 | 93.0 |
| 70 | 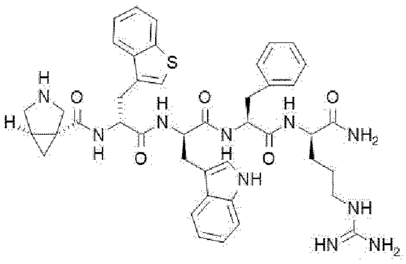 | 819 | 819.3 | 97.4 |
| 71 | 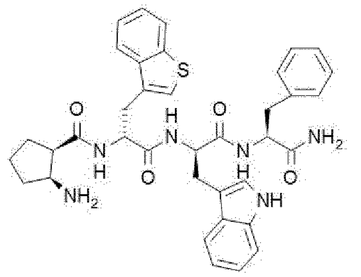 | 664.82 | 665.2 | 93.0 |
| 72 | 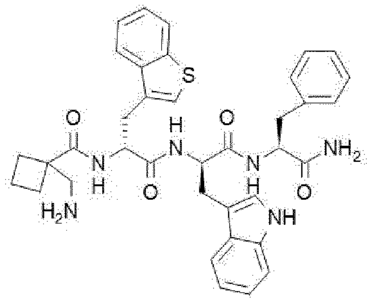 | 664.82 | 665.7 | 95.8 |

(continued)

| SEQ ID NO. | Structure | Calculated M.W. (g/mol) | ObservedM.W. (g/mol) | Purity (%) |
|---|---|---|---|---|
| 73 | 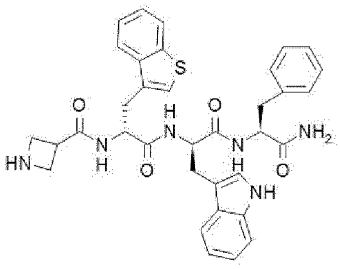 | 636.77 | 637.6 | 94.3 |
| 74 | 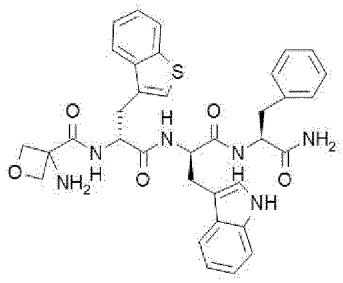 | 652.77 | 653.3 | 94.8 |
| 75 | 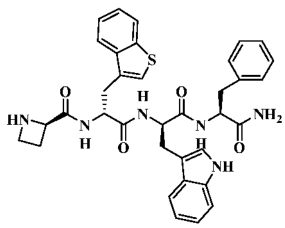 | 636.77 | 637.5 | 95.7 |
| 76 | 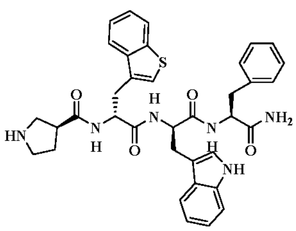 | 650.8 | 651.1 | 93.6 |
| 77 | 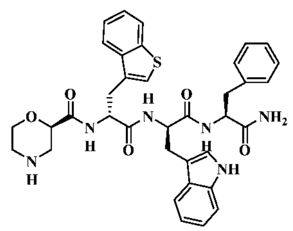 | 666.8 | 667.5 | 95.0 |
| 78 | 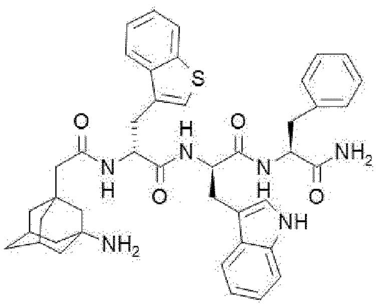 | 744.95 | 745.8 | 94.7 |

(continued)

| SEQ ID NO. | Structure | Calculated M.W. (g/mol) | ObservedM.W. (g/mol) | Purity (%) |
|---|---|---|---|---|
| 79 | 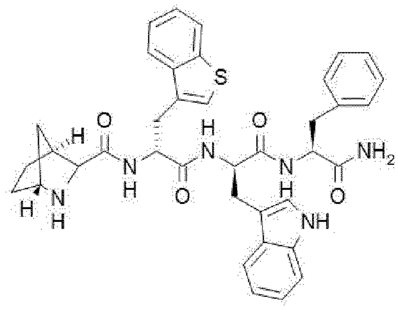 | 676.84 | 677.7 | 96.4 |
| 80 | 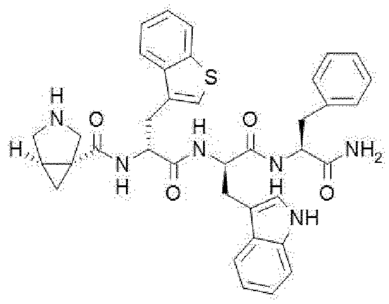 | 662.81 | 663.4 | 93.6 |

Example 4

Assessment of Agonistic Activity of Polypeptide Compounds for GHSR-la ($IC_{50}$) Screening for GHSR active compounds was accomplished by recombinant expression of the receptor. The use of recombinant expression of GHSR provides several advantages; for example, the receptor can be expressed in a determined cell system, so that it is easier to distinguish between the reactions of the compounds with GHSR and the reactions with other receptors. For example, cell lines such as HEK293, COS7 and CHO that normally express GHSR without using expression vectors can be used to express GHSR, and the same cell lines without expression vectors are used as controls.

The activity of GHSR-la can be measured using different techniques, for example, by detecting the change in the intracellular conformation of GHSR, the change in G-protein coupling activity, and/or the change in intracellular messengers. Techniques such as measuring intracellular $Ca^{2+}$ are preferably used to measure the activity of GHSR-la. Examples of techniques known in the art that can be used to measure $Ca^{2+}$ include the use of FLIPR® calcium ion assay kits, among others. The FLIPR® calcium ion assay kits use a calcium ion sensitive indicator and a masking dye to ensure that a researcher carries out high-sensitivity fluorescent screening for G protein-coupled receptors, ion channels and other calcium ion sensitive targets. This experiment used FLIPR calcium 6 assay kits and FLIPR calcium 6-QF assay kits.

1. Process

1.1. Cell culture and reagent preparation

a) Cell line: Flp In-CHO-GHSR Stable Pool;
b) Complete medium: F12K + 10% fetal bovine serum + 1× penicillin-streptomycin (PS) + 600 μg/mL hygromycin B;
c) Cell seeding medium: F12K + 10% fetal bovine serum.
d) Assay buffer: 1× HBSS + 20 mM HEPES.
e) 10× component A: Assay buffer and component A were left at room temperature (RT), 10 mL of buffer was added to component A, and the mixture was vortexed for 1-2 min and stored at -20 °C.

1.2. Compound management

a) Compound stock solutions: the powders from in-house synthesis were made into 10 mM stock solutions in DMSO according to the standard protocol.
b) Compound storage: all compounds in DMSO were stored in room temperature desiccators for short-term storage (at most 4 months). The remaining compounds were left at -20 °C for long-term storage.

1.3. Agonist activity assay

a) Flp In-CHO-GHSR Stable Pool cells were cultured in complete medium.
b) The cells were placed in 25 lbs/inch cell seeding medium in a 384-well cell culture plate (Corning, 3764) at 7k cells/well and cultured overnight at 37 °C with 5% $CO_2$.
c) 20× component A was thawed at room temperature, diluted with assay buffer to 2×, and left at RT.
d) The Petri dish was taken out of the incubator and equilibrated at room temperature for 10 min. The medium was changed to apricot buffer. After the final wash, 20 μL of buffer was kept in each well, 20 μL of 2× component A was then added to each well, and the plate was incubated at 37 °C for 3-5 s.
e) 10 μL of 5× compound was added to the 384-well cell culture plate, and data collection was immediately performed using FLIPR Tetra.

2. Data analysis

1)

$$Z' \text{ factor} = 1 - 3 \times (SD_{Max} + SD_{Min}) / (Mean_{Max} - Mean_{Min});$$

2)

$$CV_{Max} = (SD_{Max} / Mean_{Max}) \times 100\%;$$

3)

$$CV_{Min} = (SD_{Min} / Mean_{Min}) \times 100\%;$$

4) S/B = Singal/Background;
5) Calculation formula for $IC_{50}$:

$$Y = Bottom + (Top - Bottom) / (1 + 10\text{^}((LogIC_{50} - X) \times HillSlope))$$

X: log value of compound concentration; Y: Activation % or Inhibition %

According to the method described above, the activity results are shown in Table 2.

Table 2. The activity ($IC_{50}$) of polypeptide compounds for GHSR-1a

| SEQ ID NO. | $IC_{50}$ (nM) |
|---|---|
| 1 | 149.7 |
| 2 | 3.5 |

(continued)

| SEQ ID NO. | $IC_{50}$ (nM) |
|---|---|
| 3 | 17.2 |
| 4 | 1119.0 |
| 5 | >1000 |
| 6 | >1000 |
| 7 | 935.2 |
| 8 | >1000 |
| 9 | 101.3 |
| 10 | 223.2 |
| 11 | 20.3 |
| 12 | 53.7 |
| 13 | 43.5 |
| 14 | 33.2 |
| 15 | 2.3 |
| 16 | 0.7 |
| 17 | 31.1 |
| 18 | 23.5 |
| 19 | 42.6 |
| 20 | 57.4 |
| 21 | 23.0 |
| 22 | 2.3 |
| 23 | 3.3 |
| 24 | 5.4 |
| 25 | 18.1 |
| 26 | 19.5 |
| 27 | 32.8 |
| 28 | 54.1 |
| 29 | 13.3 |
| 30 | 13.4 |
| 31 | 20.8 |
| 32 | 2.4 |
| 33 | 3.1 |
| 34 | 78.4 |
| 35 | 102.3 |
| 36 | 122.1 |
| 37 | 291.3 |
| 38 | 400.5 |
| 39 | 272.2 |
| 40 | 510.3 |

(continued)

| SEQ ID NO. | $IC_{50}$ (nM) |
|---|---|
| 41 | 31.4 |
| 42 | 40.1 |
| 43 | 22.3 |
| 44 | 13.5 |
| 45 | 5.1 |
| 46 | 2.8 |
| 47 | 10.7 |
| 48 | 12.1 |
| 49 | 59.4 |
| 50 | 67.2 |
| 51 | 3.2 |
| 52 | 3.8 |
| 53 | 11.2 |
| 54 | 13.9 |
| 55 | 20.0 |
| 56 | 5.5 |
| 57 | 13.4 |
| 58 | 103.9 |
| 59 | 16.8 |
| 60 | 22.4 |
| 61 | 20.5 |
| 62 | 30.3 |
| 63 | 27.0 |
| 64 | 38.1 |
| 65 | 34.3 |
| 66 | 12.0 |
| 67 | 23.3 |
| 68 | 330.2 |
| 69 | 40.2 |
| 70 | 39.7 |
| 71 | 3.3 |
| 72 | 4.0 |
| 73 | 15.9 |
| 74 | 17.3 |
| 75 | 12.4 |
| 76 | 3.9 |
| 77 | 13.6 |
| 78 | 78.4 |

(continued)

| SEQ ID NO. | $IC_{50}$ (nM) |
|---|---|
| 79 | 33.1 |
| 80 | 35.1 |
| Ghrelin (control) | 43.1 |

As can be seen from the results in Table 2, the polypeptide compounds provided by the present invention showed agonistic activity for GHSR-la.

Example 5

Inhibition of Cytochrome P450 Oxidase by Polypeptide Compounds

Human liver microsomes containing cytochrome P450 (0.253 mg/mL protein) were incubated with test compounds (0.05-50 μM), CYPs substrates (10 μM paracetamol, 5 μM diclofenac, 30 μM mephenytoin, 5 μM dextromethorphan hydrobromide and 2 μM midazolam) and 1.0 mM NADP at 37 °C for 10 min. Naphthoflavone, sulfaphenazole, N-3-benzylnirvanol, quinidine and ketoconazole were used as reference inhibitors. The results are shown in Table 3.

Table 3. The inhibitory activity ($IC_{50}$) of compounds against cytochrome P450 CYP isoenzyme

| CYPs / Compound | 1A2 (μM) | 2D6 (μM) | 3A4 (μM) | 2C9 (μM) | 2C19 (μM) |
|---|---|---|---|---|---|
| 2 | >50 | >50 | 20.0 | >50 | >50 |
| 3 | >50 | >50 | >50 | >50 | >50 |
| 11 | >50 | >50 | 33.4 | >50 | >50 |
| 12 | >50 | >50 | >50 | >50 | >50 |
| 13 | >50 | >50 | >50 | >50 | >50 |

| | | | | | |
|---|---|---|---|---|---|
| 14 | >50 | >50 | >50 | >50 | >50 |
| 15 | >50 | 31.1 | 8.82 | >50 | >50 |
| 16 | >50 | 35.4 | 19.8 | >50 | >50 |
| 21 | >50 | >50 | >50 | >50 | >50 |
| 22 | >50 | >50 | >50 | >50 | >50 |
| 23 | >50 | >50 | >50 | >50 | >50 |
| 24 | >50 | >50 | >50 | >50 | >50 |
| 25 | >50 | >50 | >50 | >50 | >50 |
| 26 | >50 | >50 | >50 | >50 | >50 |
| 30 | >50 | >50 | >50 | >50 | >50 |
| 32 | >50 | 24.8 | 2.55 | >50 | >50 |
| 33 | >50 | >50 | >50 | >50 | >50 |
| 45 | >50 | >50 | >50 | >50 | >50 |
| 46 | >50 | >50 | >50 | >50 | >50 |
| 47 | >50 | >50 | >50 | >50 | >50 |
| 48 | >50 | >50 | >50 | >50 | >50 |
| 51 | >50 | >50 | >50 | >50 | >50 |
| 52 | >50 | >50 | >50 | >50 | >50 |
| 53 | >50 | >50 | >50 | >50 | >50 |
| 54 | >50 | >50 | >50 | >50 | >50 |
| 56 | >50 | >50 | >50 | >50 | >50 |
| 71 | >50 | >50 | >50 | >50 | >50 |
| 72 | >50 | >50 | >50 | >50 | >50 |
| 76 | >50 | >50 | >50 | >50 | >50 |
| 77 | >50 | >50 | >50 | >50 | >50 |
| 80 | >50 | >50 | >50 | >50 | >50 |

As can be seen from the results in Table 3, the inhibitory $IC_{50}$ values of the polypeptide compounds provided by the present invention against cytochrome P450 oxidase are all greater than 50 μM.

Example 6

Preparation and Activity Assays of Polypeptide Compounds 81-84

Compounds 81-84 shown in Table 4 below were prepared using the same method as 1-80 in the examples described above, and the activity ($IC_{50}$) of these compounds for GHSR was measured using the method described in Example 4. The results are shown in Table 4.

Table 4. The activity ($IC_{50}$) of polypeptide compounds for GHSR

| SEQ ID NO. | Structure | $IC_{50}$ (nM) |
|---|---|---|
| 81 | 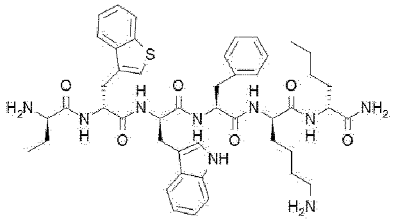 | 7.2 |
| 82 | 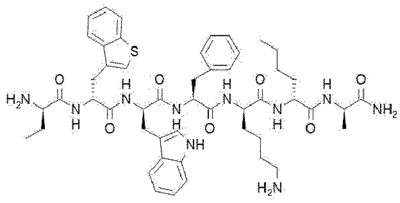 | 9.0 |
| 83 | 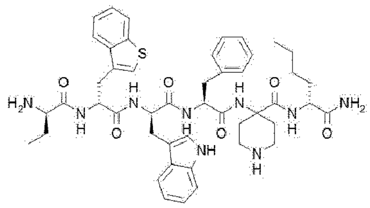 | 12.2 |
| 84 | 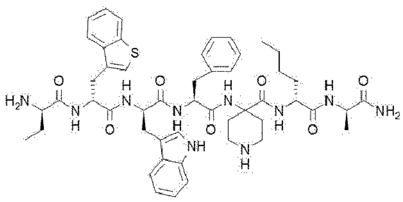 | 6.7 |
| Ghrelin (control) | | 43.1 |

As can be seen from the results in Table 4, the addition of additional amino acids to the C-termini of the pentapeptide compounds of compounds 1-80 did not significantly affect their agonistic activity for GHSR.

The above description of the examples is only intended to facilitate the understanding of the method of the present invention and its core concepts. It should be pointed out that for those skilled in the art, without departing from the principle of the present invention, several improvements and modifications can also be made to the present invention, and these improvements and modifications also fall within the protection scope of the claims of the present invention.

## Claims

1. A polypeptide compound having a structure represented by formula I, or a stereoisomer, mixture or pharmaceutically acceptable salt thereof:

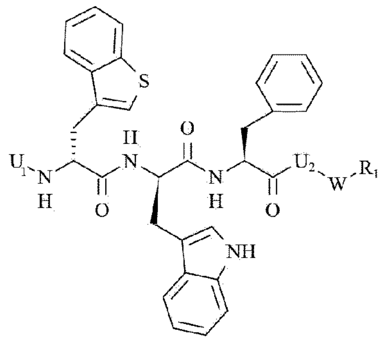

formula I;

wherein $R_1$ is selected from $-NR_2R_3$, $-OR_2$ and $-SR_2$;
and, $R_1$ is not a D- or L-amino acid;
$R_2$ and $R_3$ are independently selected from hydrogen, deuterium, a polymer derived from polyethylene glycol, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl;
W is selected from a single bond, a D-amino acid and an L-amino acid;
$U_1$ is selected from any one of the following structures:

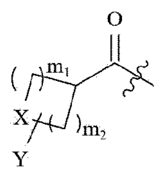 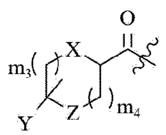 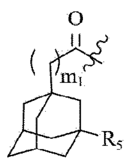 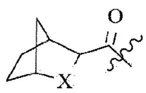 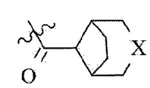 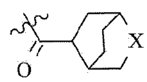

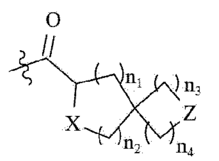 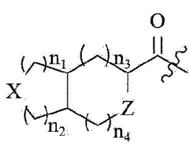 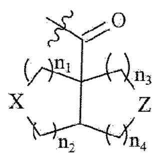 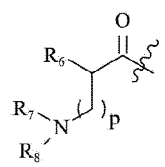 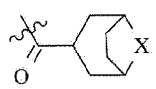 ;

wherein X and Z are independently selected from $CH-R_4$, $N-R_4$, O, S, Se, S=O and O=S=O;
$R_4$, $R_7$ and $R_8$ are independently selected from hydrogen, deuterium, amino, a protective group, a polymer derived from polyethylene glycol, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, substituted or unsubstituted aralkyl, and $R_9CO-$;
Y is selected from halogen, amino, nitro, hydroxyl and cyano;
$R_5$ is selected from $-NR_2R_3$, $-OR_2$ and $-SR_2$;
$R_6$ is selected from hydrogen, deuterium, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl;
$m_1$ and $m_2$ are independently selected from 0, 1, 2 and 3; particularly, when X is N, $m_1$ is 2, and $m_2$ is then independently selected from 0, 1 and 3; $m_2$ is 2, and $m_1$ is then independently selected from 0, 1 and 3;
$m_3$ and $m_4$ are independently selected from 0, 1, 2 and 3;
$n_1$, $n_2$, $n_3$ and $n_4$ are independently selected from 0, 1, 2 and 3;
p is 0, 1, 2, 3, 4 or 5;
$U_2$ is a single bond, or is selected from any one of the following structures, and the carbonyl end of $U_2$ is linked to W:

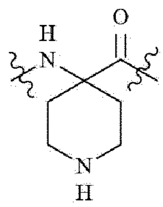 (Apc), 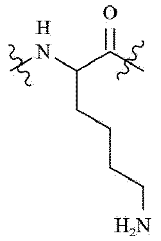 (Lys), 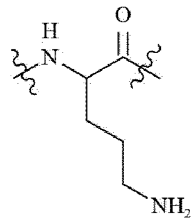 (Orn) and 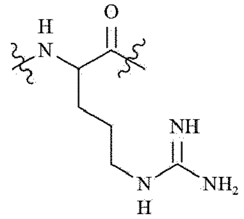

(Arg);

$R_9$ is selected from hydrogen, an acyclic substituted or unsubstituted aliphatic group, a substituted or unsubstituted alicyclic group, substituted or unsubstituted heterocyclyl, substituted or unsubstituted heteroarylalkyl, substituted or unsubstituted aryl, and substituted or unsubstituted aralkyl.

**2.** The polypeptide compound according to claim 1, wherein the $R_7$ and $R_8$ are selected from hydrogen, $C_{1-6}$ alkyl, $C_{6-14}$ aryl, $C_{3-8}$ cycloalkyl and $C_{2-10}$ acyl;
the $R_1$ is selected from $-NR_2R_3$ and $-OR_2$, wherein $R_2$ and $R_3$ are independently selected from hydrogen, methyl, ethyl, hexyl, dodecyl and hexadecyl.

**3.** The polypeptide compound according to claim 1, wherein the W is selected from one or more of a single bond and alanine, arginine, asparagine, cysteine, glutamine, aspartic acid, glutamic acid, glycine, histidine, isoleucine, leucine, lysine, methionine, phenylalanine, proline, serine, threonine, tryptophan, tyrosine and valine residues.

**4.** The polypeptide compound according to claim 1, wherein the $U_1$ is selected from the following structures:

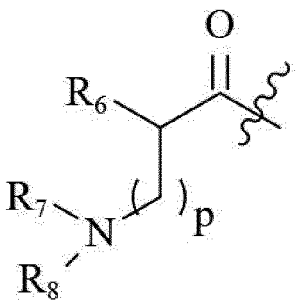 ;

wherein $R_6$ is selected from hydrogen, substituted or unsubstituted $C_{1-6}$ alkyl, substituted or unsubstituted $C_{6-14}$ aryl, and substituted or unsubstituted $C_{3-8}$ cycloalkyl; the substituted group is selected from halogen, amino, nitro, hydroxyl, acyl-substituted amino, ureido and guanidino;
$R_7$ and $R_8$ are independently selected from hydrogen, $C_{1-6}$ alkyl, $C_{6-14}$ aryl, $C_{3-8}$ cycloalkyl and $C_{2-10}$ acyl;
p is 0, 1, 2, 3, 4 or 5.

**5.** The polypeptide compound according to claim 4, wherein the $R_6$ is selected from hydrogen and substituted or unsubstituted methyl, ethyl, propyl, isopropyl, butyl, isobutyl or tert-butyl;

the substituted group is selected from halogen, amino, nitro, hydroxyl, formamido, acetamido, propionamido, butyramido, ureido and guanidino;
$R_7$ and $R_8$ are independently selected from hydrogen, methyl, ethyl, propyl, isopropyl, butyl, isobutyl, tert-butyl, formyl, acetyl, propionyl and butyryl;
p is 0, 1, 2, 3, 4 or 5.

**6.** The polypeptide compound according to claim 1, wherein the $U_1$ is selected from any one of the following structures:

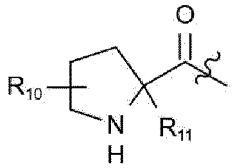, 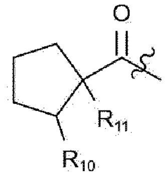, 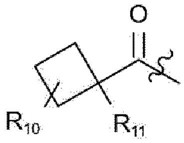, 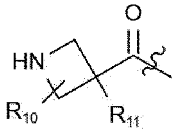, 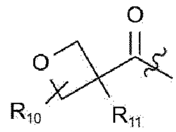,

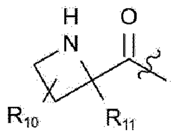, 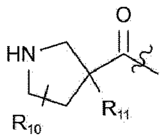, 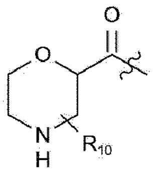, 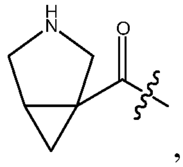, 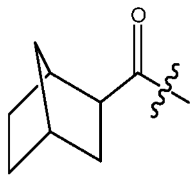,

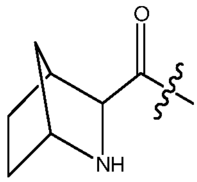 and 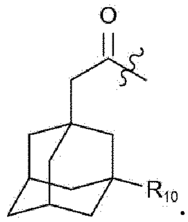;

wherein $R_{10}$ and $R_{11}$ are independently selected from hydrogen, amino, nitro, hydroxyl, halogen, cyano, aminomethyl, aminoethyl, aminopropyl and aminobutyl.

**7.** The polypeptide compound according to claim 1, having any one of the following structures, or a stereoisomer, mixture or pharmaceutically acceptable salt thereof:

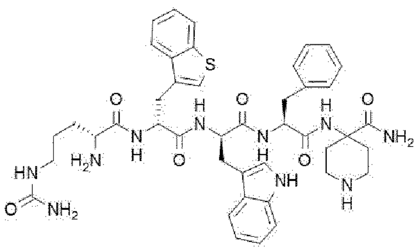, 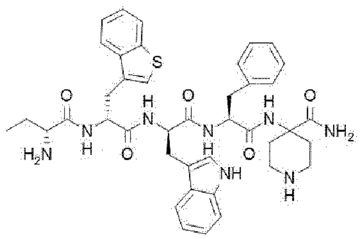, 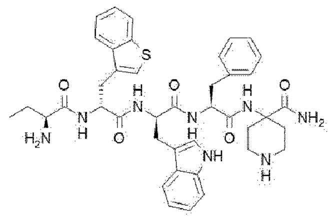,

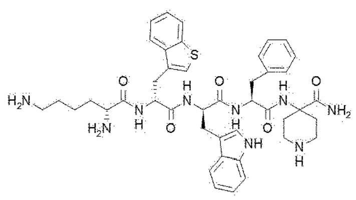, 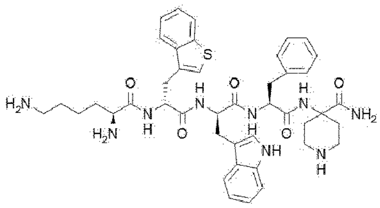, 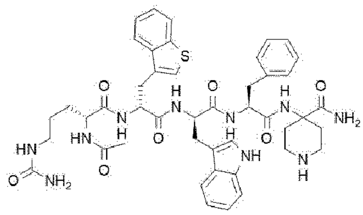,

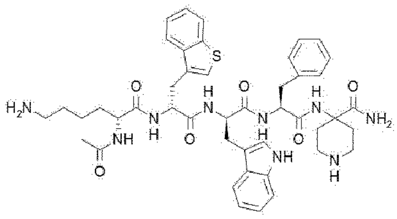, 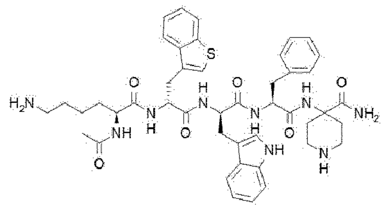, 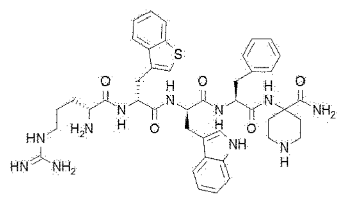,

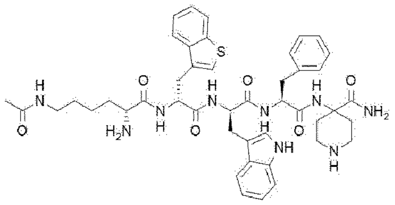,
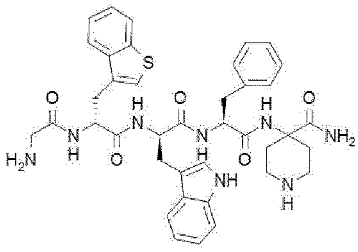,
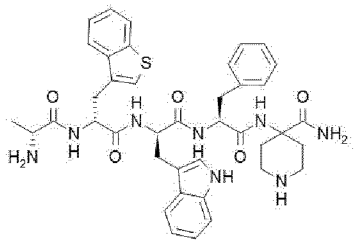,

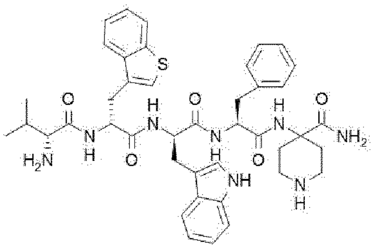,
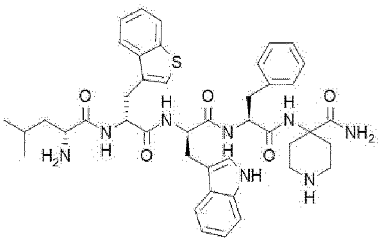,
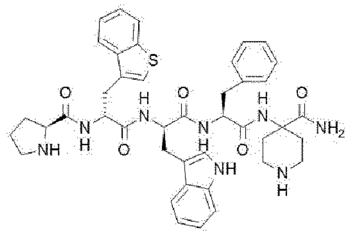,

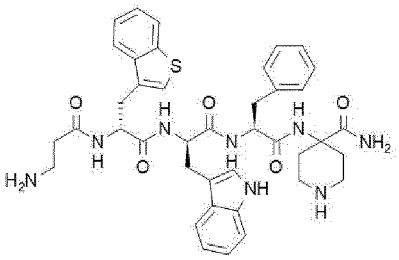,
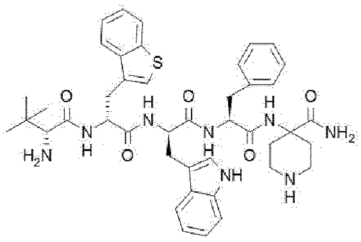,
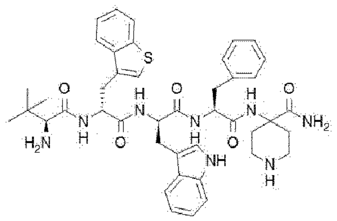,

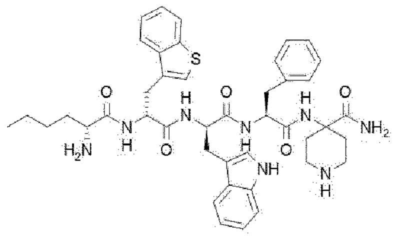,
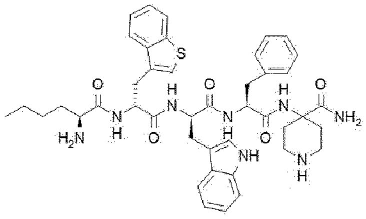,
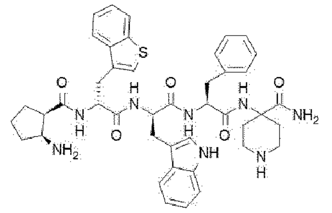,

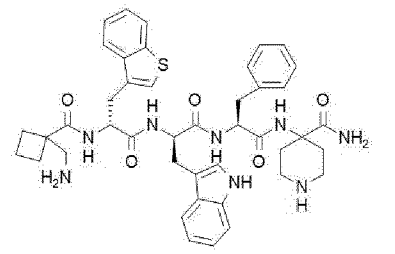,
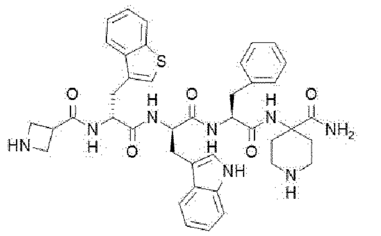,
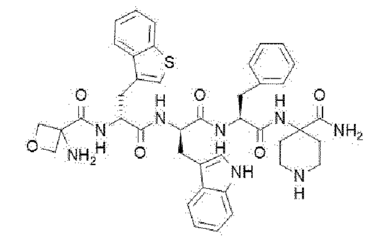,

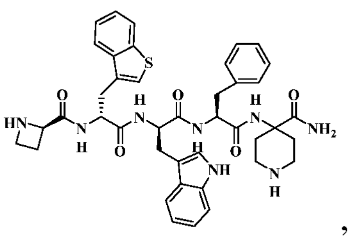,
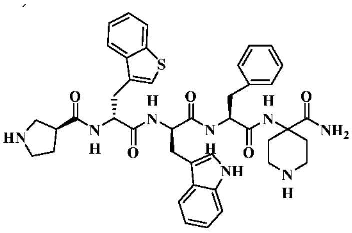,
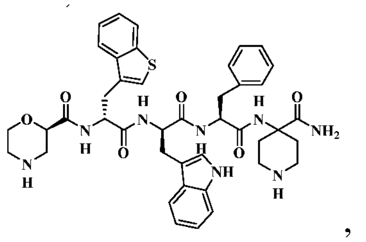,

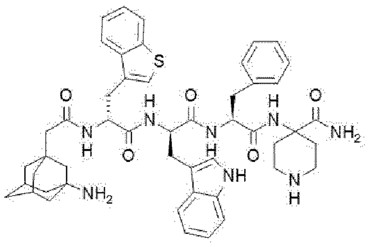 , 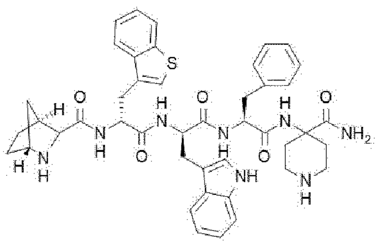 , 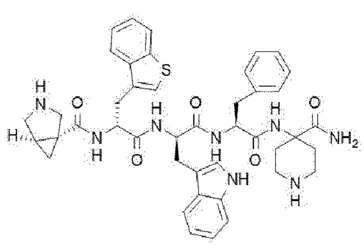 ,

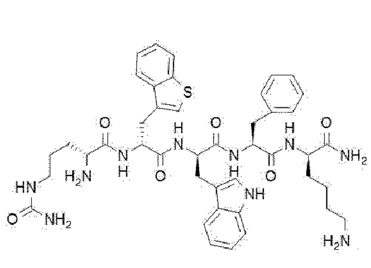 , 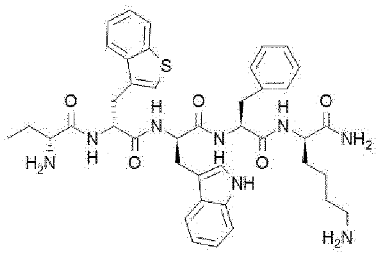 , 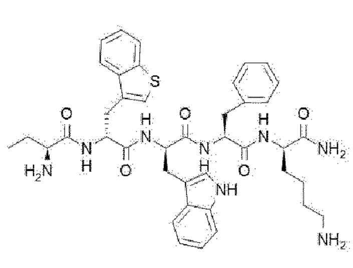 ,

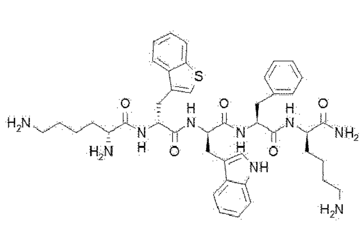 , 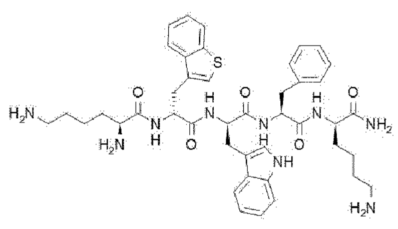 , 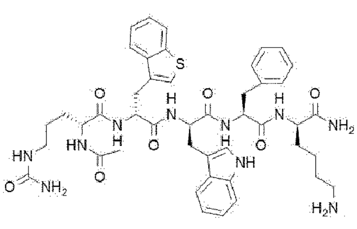 ,

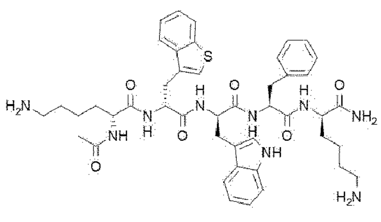 , 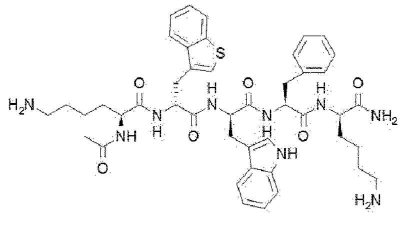 , 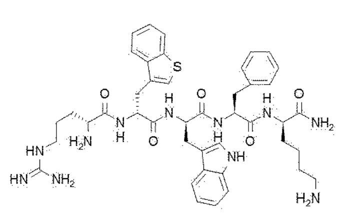 ,

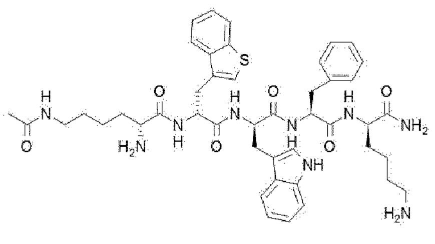 , 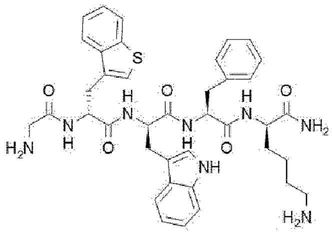 , 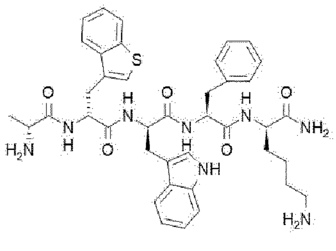 ,

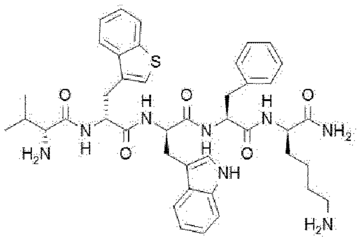 , 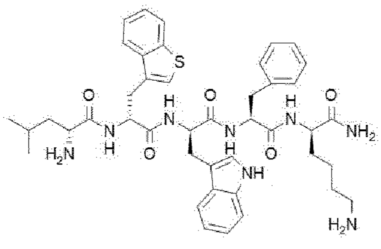 , 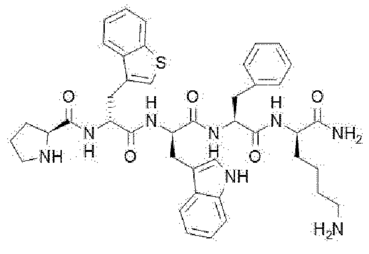 ,

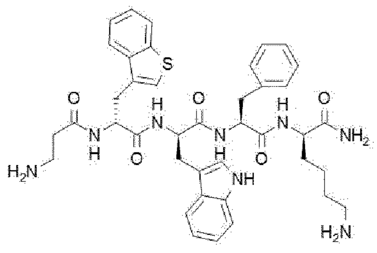,
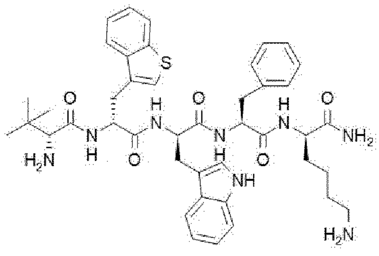,
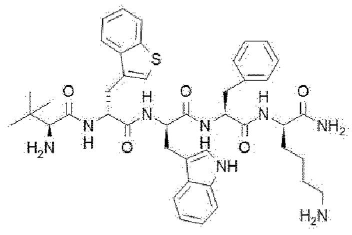,

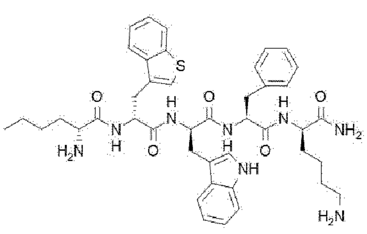,
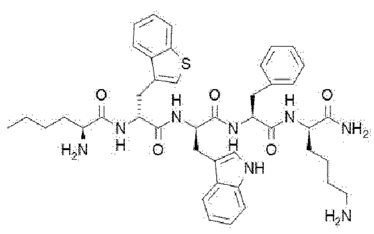,
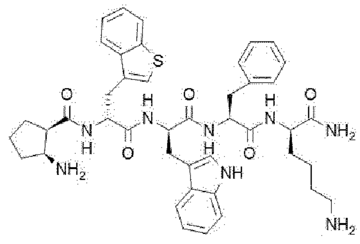,

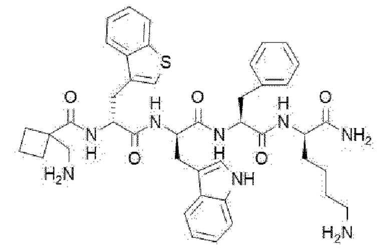,
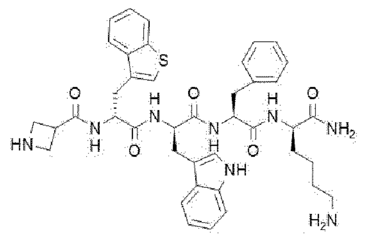,
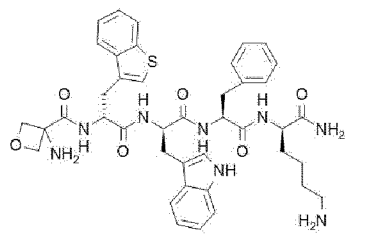,

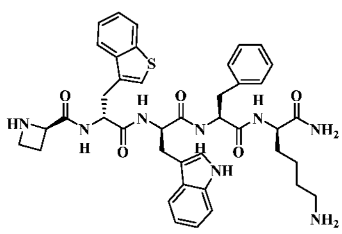,
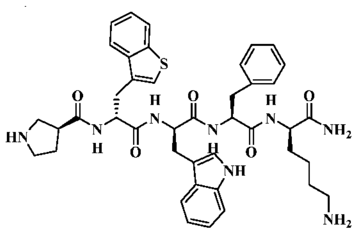,
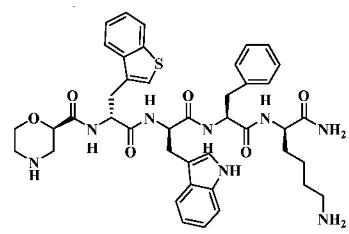,

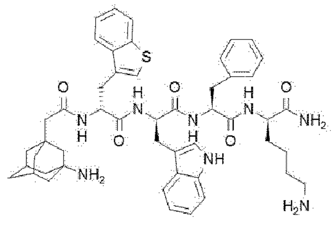,
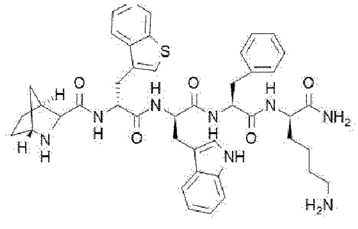,
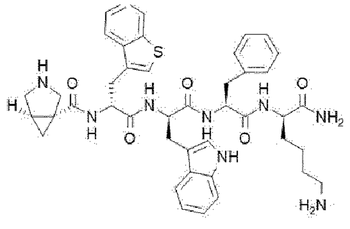,

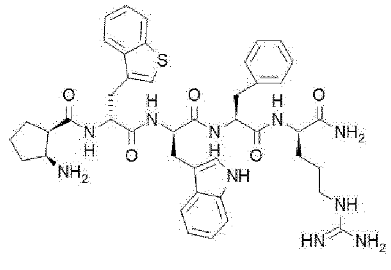,
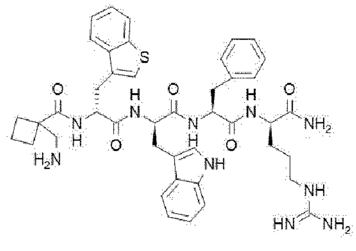,
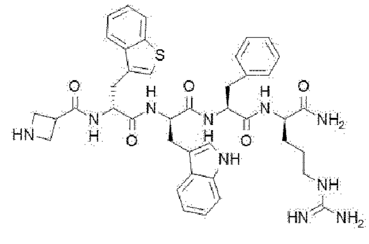,

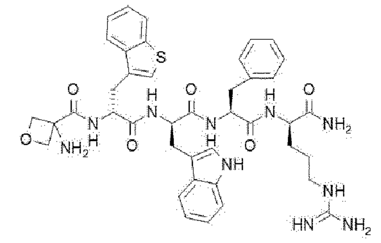,
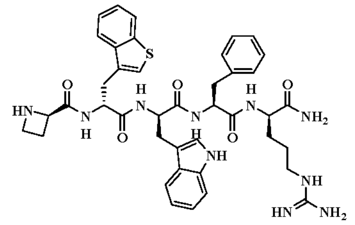,
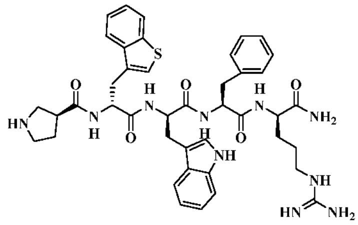,

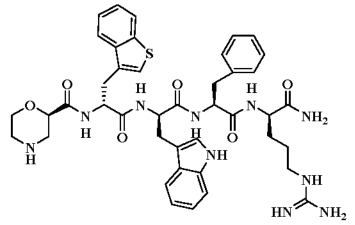,
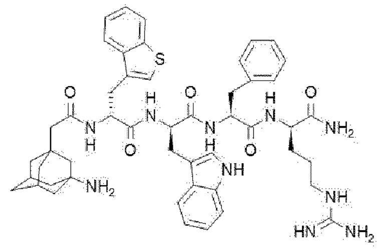,
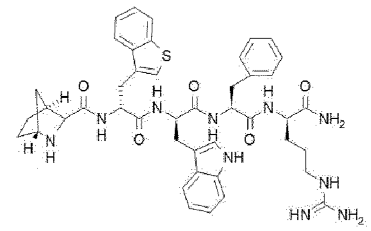,

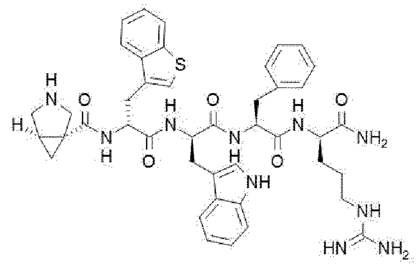,
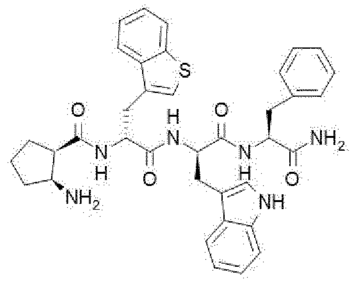,
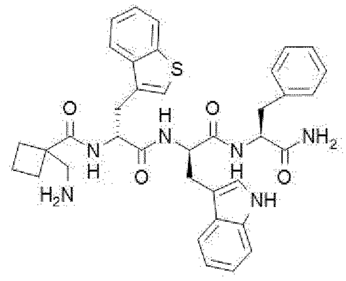,

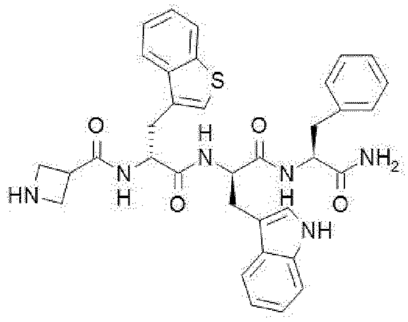,
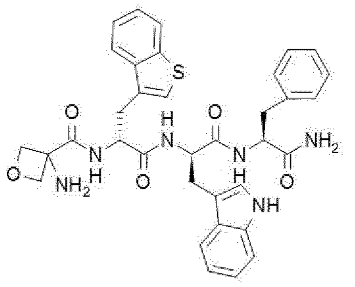,
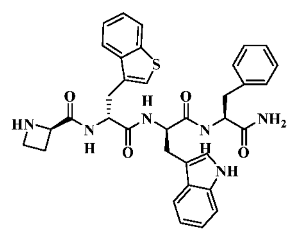,

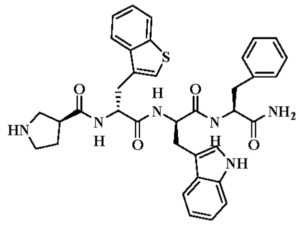,
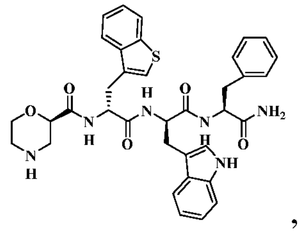,
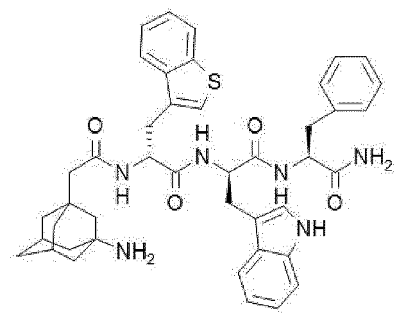,

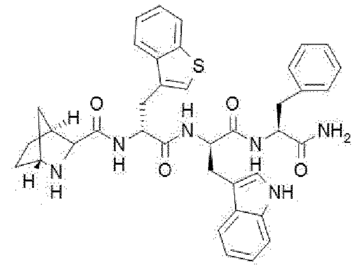,
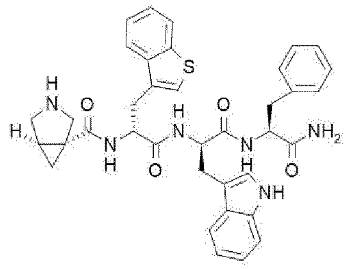,
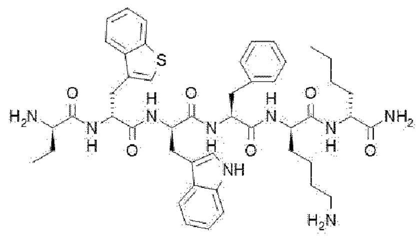,

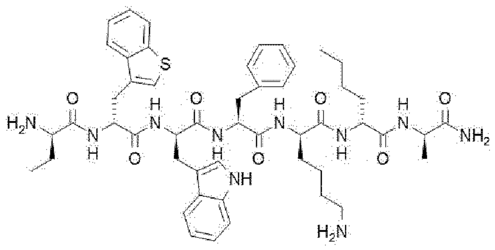,
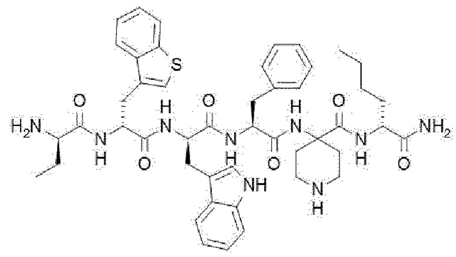

and

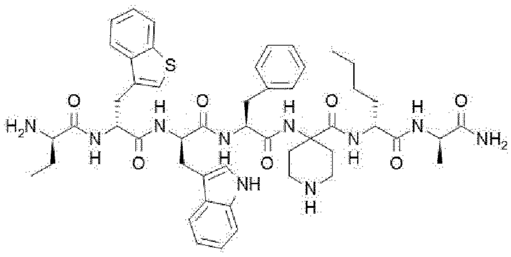.

**8.** A composition comprising the polypeptide compound according to any one of claims 1-7, and an acceptable auxiliary agent.

**9.** Use of the polypeptide compound according to any one of claims 1-7 or the composition according to claim 8 as an agonist for growth hormone secretagogue receptor, or for preparing a medicament for treating, preventing, alleviating and/or diagnosing a related disease caused by a disorder mediated by growth hormone secretagogue receptor, or as a health product for promoting growth and development.

**10.** The use according to claim 9, wherein the related disease is growth hormone deficiency.

**INTERNATIONAL SEARCH REPORT**

International application No.
**PCT/CN2022/082973**

**A. CLASSIFICATION OF SUBJECT MATTER**

C07K 7/06(2006.01)i； C07K 5/117(2006.01)i； A61K 38/08(2019.01)i； A61K 38/07(2006.01)i； A61P 5/06(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; CNKI; STNext reg/caplus: 生长激素释放肽, ghrelin, 生长激素促分泌素受体, GHSR, relamorelin, RM 131, BIM 28131, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| PX | CN 113072617 A (CHENGDU NUOHE SHENGTAI BIOTECHNOLOGY CO., LTD.) 06 July 2021 (2021-07-06)<br>claims 1-10 | 1-10 |
| PX | CN 113045625 A (CHENGDU NUOHE SHENGTAI BIOTECHNOLOGY CO., LTD.) 29 June 2021 (2021-06-29)<br>claims 1-11 | 1-10 |
| X | CN 101108875 A (SOD CONSEILS RECH APPLIC) 23 January 2008 (2008-01-23)<br>description, pp. 9-26 and 31, and claim 6 | 1-10 |
| A | WO 2007041278 A2 (SOCIETE DE CONSEILS DE RECHERCHES ETD'APPLICATIONS SCIENTIFIQUES S.A.S.) 12 April 2007 (2007-04-12)<br>entire document | 1-10 |
| A | WO 2009020643 A2 (SOCIETE DE CONSEILS DE RECHERCHES ETD'APPLICATIONS SCIENTIFIQUES S.A.S.) 12 February 2009 (2009-02-12)<br>entire document | 1-10 |
| A | WO 2017197328 A1 (LYRIC PHARMACEUTICALS INC.) 16 November 2017 (2017-11-16)<br>entire document | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 June 2022** | **21 June 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.
PCT/CN2022/082973

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **9**
because they relate to subject matter not required to be searched by this Authority, namely:

[1] Part of the technical solution of claim 9 relates to an application of a polypeptide compound as a growth hormone secretagogue receptor agonist or an application of a polypeptide compound as a health care product for promoting growth and development, and said claim does not comply with PCT Rule 39.1(iv). A search is conducted on the basis that the title of the subject matter is an application of a polypeptide compound in the preparation of a growth hormone secretagogue receptor agonist drug or in the preparation of a health care product for promoting growth and development.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/082973**

| Patent document cited in search report | Publication date (day/month/year) | Patent family member(s) | Publication date (day/month/year) |
|---|---|---|---|
| CN 113072617 A | 06 July 2021 | None | |
| CN 113045625 A | 29 June 2021 | None | |
| CN 101108875 A | 23 January 2008 | AU 2003259062 A1 | 25 February 2004 |
| | | JP 2005535707 A | 24 November 2005 |
| | | PL 373605 A1 | 05 September 2005 |
| | | US 2016311855 A1 | 27 October 2016 |
| | | SG 177006 A1 | 30 January 2012 |
| | | NO 20050205 L | 04 March 2005 |
| | | TW 200408402 A | 01 June 2004 |
| | | AR 062801 A2 | 10 December 2008 |
| | | IN 1229KOLNP2007 A | 01 August 2008 |
| | | ES 2334223 T3 | 08 March 2010 |
| | | MX PA05000974 A | 16 May 2005 |
| | | WO 2004014415 A1 | 19 February 2004 |
| | | CA 2494300 A1 | 19 February 2004 |
| | | EP 1542716 A1 | 22 June 2005 |
| | | CN 102850436 A | 02 January 2013 |
| | | RU 2007145026 A | 10 June 2009 |
| | | RU 2005106236 A | 20 January 2006 |
| | | US 2013123170 A1 | 16 May 2013 |
| | | DK 1542716 T3 | 01 February 2010 |
| | | KR 20050034716 A | 14 April 2005 |
| | | IL 165998 D0 | 15 January 2006 |
| | | US 2008242619 A1 | 02 October 2008 |
| | | DE 60329923 D1 | 17 December 2009 |
| | | BR 122016015852 B1 | 19 June 2018 |
| | | US 2015031615 A1 | 29 January 2015 |
| | | CN 1674927 A | 28 September 2005 |
| | | US 2005148515 A1 | 07 July 2005 |
| | | JP 2009149659 A | 09 July 2009 |
| | | AT 447411 T | 15 November 2009 |
| | | BR PI0313273 B1 | 12 February 2019 |
| | | US 2017362275 A1 | 21 December 2017 |
| | | US 2020017548 A1 | 16 January 2020 |
| | | AR 040965 A1 | 27 April 2005 |
| | | CZ 20041254 A3 | 13 April 2005 |
| | | BR 0313273 A | 05 July 2005 |
| | | EP 2130548 A2 | 09 December 2009 |
| WO 2007041278 A2 | 12 April 2007 | PT 1937262 T | 21 August 2019 |
| | | CN 103251933 A | 21 August 2013 |
| | | US 2009304724 A1 | 10 December 2009 |
| | | JP 2013035856 A | 21 February 2013 |
| | | RU 2011103062 A | 10 August 2012 |
| | | SI 1937262 T1 | 30 September 2019 |
| | | JP 2015038116 A | 26 February 2015 |
| | | US 2019248861 A1 | 15 August 2019 |
| | | US 2017037104 A1 | 09 February 2017 |
| | | ES 2740108 T3 | 05 February 2020 |
| | | CA 2625447 A1 | 12 April 2007 |
| | | JP 2009510098 A | 12 March 2009 |

Form PCT/ISA/210 (patent family annex) (January 2015)

**INTERNATIONAL SEARCH REPORT**
**Information on patent family members**

International application No.
**PCT/CN2022/082973**

| Patent document cited in search report | Publication date (day/month/year) | Patent family member(s) | Publication date (day/month/year) |
|---|---|---|---|
| | | US 2020347110 A1 | 05 November 2020 |
| | | EP 3586846 A1 | 01 January 2020 |
| | | DK 1937262 T3 | 15 July 2019 |
| | | PL 1937262 T3 | 31 October 2019 |
| | | EP 1937262 A2 | 02 July 2008 |
| | | US 2011166069 A1 | 07 July 2011 |
| | | RU 2008116837 A | 10 November 2009 |
| | | US 2015218245 A1 | 06 August 2015 |
| | | CN 105381453 A | 09 March 2016 |
| | | CN 101287464 A | 15 October 2008 |
| | | HU E044391 T2 | 28 October 2019 |
| | | JP 2017095494 A | 01 June 2017 |
| WO 2009020643 A2 | 12 February 2009 | CA 2921685 A1 | 12 February 2009 |
| | | AR 068058 A1 | 04 November 2009 |
| | | US 2020360483 A1 | 19 November 2020 |
| | | US 2011160133 A1 | 30 June 2011 |
| | | JP 2010535770 A | 25 November 2010 |
| | | EP 2185200 A2 | 19 May 2010 |
| | | JP 2013241445 A | 05 December 2013 |
| | | JP 2018138586 A | 06 September 2018 |
| | | TW 200916113 A | 16 April 2009 |
| | | CA 2695713 A1 | 12 February 2009 |
| | | AU 2008284269 A1 | 12 February 2009 |
| | | US 2015111823 A1 | 23 April 2015 |
| | | US 2016206700 A1 | 21 July 2016 |
| | | US 2017304403 A1 | 26 October 2017 |
| WO 2017197328 A1 | 16 November 2017 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110343062


### Non-patent literature cited in the description


- **BOWERS.** *Endocrinology,* 1984, vol. 114, 1537-1545
- **STEWART J. M. ; YOUNG J. D.** Solid Phase Peptide Synthesis. Pierce Chemical Company, 1984
- **BODANZSKY M. ; BODANZSKY A.** The practice of Peptide Synthesis. Springer Verlag, 1994
- **LLOYD WILLIAMS P. et al.** Chemical Approaches to the Synthesis of Peptides and Proteins. CRC, 1997
- **KULLMANN W.** Proteases as catalysts for enzymic syntheses of opioid peptides. *J. Biol. Chem.,* 1980, vol. 255 (17), 8234-8238
- **LLOYD-WILLIAMS P. et al.** Convergent Solid-Phase Peptide Synthesis. *Tetrahedron,* 1993, vol. 49 (48), 11065-11133
- **ATHERTON B. ; SHEPPARD R. C.** Solid Phase Peptide Synthesis: A practical approach. IRL Oxford University Press, 1989
- **MATSUEDA G. R. et al.** A p-methyl benzhydrylamine resin for improved solid-phase synthesis of peptide amides. *Peptides,* 1981, vol. 2, 4550
- **BARLOS K. et al.** Darstellung geschützter Peptid-Fragmente unter Einsatz substituierter Triphenylmethyl Harze. *Tetrahedron Lett.,* 1989, vol. 30, 3943-3946
- **BARLOS K. et al.** Veresterung von partiell geschützten PeptidFragmenten mit Harzen Einsatz von 2-Chlorotritylchlorid zur Synthese von LeulGastrin I. *Tetrahedron Lett.,* 1989, vol. 30, 39473951
- **ALBERICIO F. et al.** Preparation and application of the 5-(4-(9-fluorenylmethyloxycarbonyl)aminomethyl-3,5-dimethoxy-phenoxy)valeric acid (PAL) handle for the solid-phase synthesis of C-terminal peptide amides under mild conditions. *J. Org. Chem.,* 1990, vol. 55, 3730-3743
- **RINK H.** Solid-phase synthesis of protected peptide fragments using a trialkoxy-diphenyl-methylester resin. *Tetrahedron Lett.,* 1987, vol. 28, 3787-3790
- **WANG S.S.** p-Alkoxybenzyl Alcohol Resin and p-Alkoxybenzyl oxycarbonylhydrazide Resin for Solid Phase Synthesis of Protected Peptide Fragments. *J. Am. Chem. Soc,* 1973, vol. 95, 1328-1333
- **BERGE S. M. et al.** Pharmaceutical Salts. *J. Pharm. Sci,* 1977, vol. 66, 119